# EUROPEAN PATENT SPECIFICATION

(11) **EP 3 380 059 B1**
(45) Date of publication and mention of the grant of the patent: **06.05.2020**
(21) Application number: 16816489.5
(22) Date of filing: 23.11.2016
(51) Int. Cl.: A61J 1/20

(54) **SYRINGE FILL ADAPTER**
SPRITZENFÜLLADAPTER
ADAPTATEUR DE REMPLISSAGE DE SERINGUE

(30) Priority: 25.11.2015 US 201562259906 P
(43) Date of publication of application: 03.10.2018
(73) Proprietor: Bayer Healthcare LLC, Whippany, NJ 07981 (US)
(72) Inventor: COWAN, Kevin P., Allison Park, Pennsylvania 15101 (US); DEDIG, James A., Pittsburgh, Pennsylvania 15220 (US); SCHRAUDER, Matthew, Mars, Pennsylvania 16046 (US); SPOHN, Michael A., Fenelton, Pennsylvania 16034 (US); MCDERMOTT, Michael, Pittsburgh, Pennsylvania 15237 (US); MCGEE, Matthew, Plymouth Meeting, Pennsylvania 19462 (US)
(74) Representative: BIP Patents
(86) International application number: PCT/US2016/063461
(87) International publication number: WO 2017/091643

(56) References cited:
- US-A1- 2004 186 457
- US-A1- 2004 186 457
- US-A1- 2006 149 213
- US-A1- 2006 149 213
- US-B1- 9 173 995

## Description

### CROSS-REFERENCE TO RELATED APPLICATIONS

This application claims priority to United States Provisional Patent Application No. 62/259,906, entitled "Syringe Fill Adapter" and filed on November 25, 2015.

### BACKGROUND OF THE DISCLOSURE

### Field of the Disclosure

The present disclosure relates generally to syringes, fill adapters, and syringe and fluid transfer assemblies for use in fluid delivery systems, and, more particularly, to syringes, fill adapters, and syringe and fluid transfer assemblies for use in medical fluid delivery systems in which fluids are delivered to a patient under time constraints.

### Description of the Related Art

In many medical procedures, such as drug delivery, it is desirable to inject a liquid into a patient. Numerous types of liquids, such as contrast media (often referred to simply as "contrast") and/or saline, may be injected into a patient during diagnostic and therapeutic procedures. In some medical procedures, for example, angiography, computed tomography (CT), ultrasound, magnetic resonance imaging (MRI), nuclear medicine, and positron emission tomography (PET), it is necessary to deliver a liquid, such as contrast, in a timed fashion under pressure. Injectors suitable for these applications typically use a relatively large volume syringe and are capable of producing relatively large flow rates.

Medical personnel work under increasingly difficult time and physical constraints. Thus, it is desirable to fill syringes or other liquid containers and to connect and disconnect fluid delivery systems as quickly as possible. However, filling a large syringe with liquid, such as contrast, is typically a time consuming process. Conventional syringes have a distal opening that is typically used for filling the interior of the syringe with liquid. The size of this distal opening places significant constraints on the filling rate. Further, since conventional syringes are typically shipped with the plunger in the fully retracted position, filling a syringe first requires moving the plunger to distal end of the syringe to eject air from the syringe and start the liquid filling process. Since the cost of many medical processes, such as diagnostic imaging, increases in relation to duration, any delays can significantly increase cost. US9173995 B1 discloses an example for a large syringe including a barrel having a proximal end, a distal end, and a sidewall extending between the proximal end and the distal end along a longitudinal axis. At least one syringe retaining member protrudes radially outwardly relative to an outer surface of the sidewall. The at least one syringe retaining member tapers axially in a direction from the distal end toward the proximal end. The at least one syringe retaining member is configured for selective engagement with a locking mechanism on a fluid injector to releasably lock the syringe with the fluid injector. A taper of the at least one syringe retaining member is configured to rotationally guide the syringe into alignment with the locking mechanism.

Furthermore, in many such fluid delivery systems, it is necessary to form a fluid connection between separate fluid path components. For example, it may be necessary to connect an injector-powered syringe to flexible plastic tubing that, in turn, is connected to a catheter inserted into a patient. A common connector used in the medical arts is the luer connector or luer lock. The luer connector includes a male connector or member and a female connector or member. The male member and female member are typically connected via radially inwardly projecting threading attached to the female member, which cooperates with one or more radially outwardly extending flanges on the male luer member to create a leak-free connection.

Medical personnel must connect and/or disconnect fluid delivery elements in a relatively short time and under stressed and/or emergency conditions. It is thus desirable to develop syringe adapters that are configured for filling a syringe and that have durable syringe and connector interfaces capable of connecting or disconnecting simply and quickly.

Connections between syringes and other containers are know from the prior art as described below:
US20040186457 A1 discloses a fluid flow control device adapted to control the flow of fluid from a fluid container to a fluid extraction device without the need for a needle. The fluid flow control device includes a connector element and a container insert member which define a fluid channel. A compressible valve element is disposed in the fluid channel of the connector element and the container insert member. The valve element includes a plug and shaft and resiliently prevents the flow of fluid by forming a fluid seal in the fluid channel. The valve element may be operationally engaged by the fluid extraction device thereby breaking the fluid seal and allowing the fluid to flow from the container to the fluid extraction device. However, the fluid is not directed to flow down an inner sidewall of the fluid extraction device.
US 2006/0149213 A1 discloses a self-sealing male connector device for connection with a female Luer connector. The device has an elongated male body configured with lengthwise relatively rigid and flexible wall segments cooperating to allow the body to be radially compressed from an expanded configuration to a contracted configuration. A closure cap formed with a resealable aperture is disposed on the distal end of the male body so as to be responsive to the compression of the male body. The relatively flexible wall segments may be installed within notches in the male body or be formed integral with the relatively rigid wall segments.

### SUMMARY OF THE DISCLOSURE

In general, the present disclosure relates to syringes and fill adapters used in fluid transfer assemblies for medical fluid delivery systems.

The present invention provides a fill adapter for delivery of a medical liquid to a container according to claim 1.

In accordance with other examples of the present disclosure, each of the one or more spokes may be resiliently elastic such that the flow controller is movable in a direction of the longitudinal axis of the body with the flow of the liquid. The curved distal surface of the flow controller may be convex. The curved distal surface may have a flow diverter extending distally from a central portion of the curved distal surface. The flow diverter may be shaped to direct fluid flowing through the central bore in a radially outward direction toward the gap. The flow controller may have a curved proximal surface. The curved proximal surface of the flow controller may be convex. The body may have a flange at the distal end, the flange extending radially outward relative to an outer surface of the body. At least a portion of the body may be configured to be removably received within an open distal end of the container.

In accordance with other examples of the present disclosure, a fluid transfer assembly may have a syringe for receiving a medical liquid therein, and a fill adapter. The syringe may have a proximal end, a distal end having an open-ended syringe neck, and a sidewall extending between the proximal end and the distal end along a longitudinal axis. The syringe may define an interior volume for receiving the medical liquid therein. The fill adapter may be received within the open-ended syringe neck.

In accordance with other examples of the present disclosure, an outer portion of the syringe neck may have a flange extending around at least a portion of a circumference of the syringe neck. At least a portion of the flange may be configured to engage a cap for enclosing the distal end of the syringe. The syringe may have a drive member engagement portion protruding proximally from an end wall enclosing the proximal end and configured for engagement with a drive member of a fluid injector. The sidewall of the syringe may be flexible and roll upon itself when acted upon by a drive member of a fluid injector such that an outer surface of the sidewall is folded in a radially inward direction as the drive member is advanced from the proximal end to the distal end and wherein the outer surface of the sidewall is unfolded in a radially outward direction as the drive member is retracted from the distal end to the proximal end.

In accordance with other examples of the present disclosure, a fluid transfer assembly may have a syringe for receiving a medical liquid therein, the syringe having a proximal end, a distal end having an open-ended syringe neck, and a sidewall extending between the proximal end and the distal end along a longitudinal axis. The syringe may define an interior volume for receiving the medical liquid therein. The fluid transfer assembly may further have a fill adapter received within the open-ended syringe neck. The fluid transfer assembly may further have a cap secured to the syringe neck, the cap having a nozzle in fluid communication with the interior volume of the rolling diaphragm syringe. An outer portion of the syringe neck may have a flange extending around at least a portion of a circumference of the syringe neck, and the cap may have one or more tabs configured to releasably engage at least a portion of the flange. The one or more tabs may have a first end connected to the cap and a second end extending proximally from the first end. The second end may be deflectable in a radially outward direction when the cap contacts the flange on the syringe neck.

These and other features and characteristics of the syringes, fill adapter, and syringe and fluid transfer assembly for use in medical fluid delivery systems and combination of parts and economies of manufacture will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only, and are not intended as a definition of the limits of the disclosure.

### BRIEF DESCRIPTION OF THE DRAWINGS

where only figures 30A to 34B are showing the invention as presently claimed.
**FIG. 1** is an exploded side view of a syringe and fluid transfer assembly in accordance with one example of the present disclosure;
**FIG. 2A** is a side cross-sectional view of a syringe for use with a fill adapter in accordance with one example of the present disclosure;
**FIG. 2B** is a perspective view of a syringe for use with a fill adapter in accordance with another example of the present disclosure;
**FIG. 3A** is a side view of the syringe and fluid transfer assembly of **FIG. 1****;**
**FIG. 3B** is a longitudinal cross-sectional view of **FIG. 3A****;**
**FIG. 4** is a side view of the syringe and fluid transfer assembly of **FIG. 3A** in a first engagement position;
**FIG. 5** is a side view of the syringe and fluid transfer assembly of **FIG. 3A** in a second engagement position;
**FIG. 6** is a longitudinal cross-sectional view of **FIG. 5****;**
**FIG. 7** is a side view of the syringe and fluid transfer assembly of **FIG. 1** after the syringe is filled with fluid and a piercing device has been disengaged from a fill adapter;
**FIG. 8** is a longitudinal cross-sectional view of **FIG. 7****;**
**FIG. 9** is a side view of the syringe of **FIG. 7****;**
**FIG. 10** is a longitudinal cross-sectional view of **FIG. 9****;**
**FIG. 11** is a detailed, cross-sectional view of a fill adapter of **FIG. 7****;**
**FIG. 12** is a perspective view of a syringe and fluid transfer assembly in accordance with another example of the present disclosure;
**FIG. 13** is a detailed side cross-sectional view of a connection interface of the fill adapter of **FIG. 12**;
**FIG. 14** is a detailed side cross-sectional view of a fluid transfer assembly in accordance with another example of the present disclosure;
**FIG. 15A** is a perspective view of a fluid transfer assembly in accordance with another example of the present disclosure;
**FIG. 15B** is another perspective view of the fluid transfer assembly of **FIG. 15A****;**
**FIG. 16** is an exploded perspective view of a fluid transfer assembly in accordance with another example of the present disclosure;
**FIG. 17A** is a perspective view of a fluid transfer assembly in accordance with another example of the present disclosure;
**FIG. 17B** is a detailed side cross-sectional view of the fluid transfer assembly of **FIG. 17A**;
**FIG. 18A** is a perspective view of a fluid transfer assembly in accordance with another example of the present disclosure;
**FIG. 18B** is another perspective view of the fluid transfer assembly of **FIG. 18A****;**
**FIG. 18C** is a detailed side cross-sectional view of the fluid transfer assembly of **FIG. 18A****;**
**FIG. 18D** is a detailed side cross-sectional view of a spike adapter of **FIG. 18A****;**
**FIG. 18E** is a detailed side cross-sectional view of a connection interface of the fill adapter of **FIG. 18A****;**
**FIG. 19A** is a perspective view of a fluid transfer assembly in accordance with another example of the present disclosure;
**FIG. 19B** is another perspective view of the fluid transfer assembly of **FIG. 19A****;**
**FIG. 20** is a perspective view of a fluid transfer assembly in accordance with another example of the present disclosure;
**FIG. 21A** is a detailed side cross-sectional view of a connection interface of a fill adapter in accordance with another aspect;
**FIG. 21B** is a detailed perspective cross-sectional view of the connection interface of **FIG. 21A**;
**FIG. 21C** is a perspective view of a syringe in accordance with the fill adapter of **FIG. 21A**;
**FIG. 21D** is a perspective view of a ribbed connector of a fill adapter of **FIG. 21A**;
**FIG. 21E** is a perspective view of a flow controller of the fill adapter of **FIG. 21A**;
**FIG. 21F** is a perspective view of a ribbed connector in accordance with another example of the fill adapter of **FIG. 21A**;
**FIG. 22A** is a perspective view of a fluid transfer assembly in accordance with another aspect;
**FIG. 22B** is a detailed side cross-sectional view of the fluid transfer assembly of **FIG. 22A**;
**FIG. 22C** is a detailed side cross-sectional view of a connection interface of the fill adapter of **FIG. 22A**;
**FIG. 22D** is an exploded view of a valve housing of the fluid transfer assembly of **FIG. 22A**;
**FIG. 23A** is a perspective view of a fluid transfer assembly in accordance with another example of the present disclosure;
**FIG. 23B** is a detailed side cross-sectional view of a connection interface of the fill adapter of **FIG. 23A**;
**FIG. 23C** is a perspective view of a spike adapter of the fluid transfer assembly of **FIG. 23A**;
**FIG. 23D** is a perspective view of a flow controller in accordance with one aspect of the fill adapter of **FIG. 23A**;
**FIG. 24A** is a detailed side cross-sectional view of a connection interface in accordance with another example of the fill adapter of **FIG. 23A****;**
**FIG. 24B** is a perspective view of a flow controller of the connection interface of **FIG. 39A;**
**FIG. 25A** is a detailed side cross-sectional view of a connection interface in accordance with another example of the fill adapter of **FIG. 23A****;**
**FIG. 25B** is a perspective view of a flow controller of the connection interface of FIG. **25A**;
**FIG. 26A** is a detailed side cross-sectional view of a connection interface in accordance with another aspect of the fill adapter of **FIG. 23A**;
**FIG. 26B** is a perspective view of a flow controller of the connection interface of **FIG. 26A**;
**FIG. 27A** is a perspective view of a fluid transfer assembly in accordance with another example of the present disclosure;
**FIG. 27B** is a detailed side cross-sectional view of a connection interface of the fill adapter of **FIG. 27A**;
**FIG. 27C** is another perspective view of a fluid transfer assembly of **FIG. 27A**;
**FIG. 27D** is a detailed side cross-sectional view of a connection interface of the fill adapter of **FIG. 27C**;
**FIG. 27E** is a perspective view of a spool valve of the fluid transfer assembly of **FIG. 27A**;
**FIG. 27F** is an exploded perspective view of the spool valve of **FIG. 27E**;
**FIG. 27G** is a detailed side cross-sectional view of the spool valve of **FIG. 27E**;
**FIG. 28A** is a perspective view of a fluid transfer assembly in accordance with another example of the present disclosure;
**FIG. 28B** is a detailed side cross-sectional view of a connection interface of the fill adapter of **FIG. 28A**;
**FIG. 29** is an exploded perspective view of a syringe assembly in accordance with another example of the present disclosure;
**FIG. 30A** is a perspective view of a syringe with a fill adapter in accordance with another example of the present disclosure;
**FIG. 30B** is a detailed top perspective view of the syringe and fill adapter of **FIG.** 30A;
**FIG. 30C** is a side cross-sectional view of the syringe and fill adapter of **FIG. 30A**;
**FIG. 30D** is a top perspective view of the fill adapter shown in **FIG. 30A** removed from the syringe;
**FIG. 31A** is a side cross-sectional view of a syringe and a fill adapter in accordance with another example of the present disclosure;
**FIG. 31B** is a top perspective view of the fill adapter shown in **FIG. 31A** removed from the syringe;
**FIG. 32A** is an exploded perspective view of a syringe, fill adapter, and cap in accordance with another example of the present disclosure;
**FIG. 32B** is a perspective view of the syringe, fill adapter, and cap of **FIG. 32A** in assembled form;
**FIG. 32C** is a side cross-sectional view of the syringe, fill adapter, and cap of **FIG. 32A**;
**FIG. 32D** is a perspective view of the syringe of **FIG. 32A**;
**FIG. 33A** is an exploded perspective view of a syringe, fill adapter, and cap in accordance with another example of the present disclosure;
**FIG. 33B** is a perspective view of the syringe of **FIG. 33A**;
**FIG. 33C** is a perspective view of the cap of **FIG. 33A**;
**FIG. 34A** is a side cross-sectional view of a syringe and a fill adapter in accordance with another example of the present disclosure, showing a flow controller in a closed position; and
**FIG. 34B** is a side cross-sectional view of the syringe and the fill adapter of **FIG. 34A**, showing the flow controller in an open position.

### DETAILED DESCRIPTION OF THE DISCLOSURE

The illustrations generally show non-limiting examples of the present disclosure. While the description presents various examples, it should not be interpreted in any way as limiting the disclosure. Furthermore, modifications, concepts, and applications of the disclosure's examples are to be interpreted by those skilled in the art as being encompassed, but not limited to, the illustrations and description provided herein. Any and all such modifications, variations, equivalents, and alternatives are intended to fall within the spirit and scope of the present disclosure.

As used in the specification and the claims, the singular form of "a", "an", and "the" include plural referents unless the context clearly dictates otherwise.

For purposes of the description hereinafter, the terms "upper", "lower", "right", "left", "vertical", "horizontal", "top", "bottom", "lateral", "longitudinal", and derivatives thereof shall relate to the components as they are oriented in the drawing figures. When used in relation to a syringe, the term "proximal" refers to a portion of a syringe nearest to an injector when a syringe is oriented for connecting to an injector. The term "distal" refers to a portion of a syringe farthest away from an injector when a syringe is oriented for connecting to an injector. The term "radial" refers to a direction in a cross-sectional plane normal to a longitudinal axis of a syringe extending between proximal and distal ends. The term "circumferential" refers to a direction around an inner or outer surface of a sidewall of a syringe. The term "axial" refers to a direction along a longitudinal axis of a syringe extending between the proximal and distal ends. The term "flexible", when used in connection with a syringe, means that at least a portion of a syringe, such as a sidewall of a syringe, is capable of bending or being bent to change a direction in which it extends. The terms "roll over", "rolling over", and "rolls upon itself' refer to an ability of a first portion of a syringe, such as a proximal portion of a sidewall of a syringe, to bend approximately 180° relative to a second portion of a syringe, such as a distal portion of a sidewall of a syringe, when urged by a drive member of a fluid injector.

Unless otherwise indicated, all ranges or ratios disclosed herein are to be understood to encompass any and all subranges or subratios subsumed therein. For example, a stated range or ratio of "1 to 10" should be considered to include any and all subranges between (and inclusive of) the minimum value of 1 and the maximum value of 10; that is, all subranges or subratios beginning with a minimum value of 1 or more and ending with a maximum value of 10 or less, such as but not limited to, 1 to 6.1, 3.5 to 7.8, and 5.5 to 10.

It is to be understood that the specific devices and processes illustrated in the attached drawings, and described in the following specification, are simply exemplary aspects of the disclosure. Hence, specific dimensions and other physical characteristics related to the aspects disclosed herein are not to be considered as limiting.

Referring to the drawings in which like reference characters refer to like parts throughout the several views thereof, syringes, fill adapters, and syringe and fluid transfer assemblies for use in medical fluid delivery systems will be described herein in detail. The present disclosure also provides other connectors suitable for use with the syringes disclosed herein, as well as with other fluid path elements or fluid pumping systems. In general, the connectors of the present disclosure are suitable for use in low-pressure and high-pressure fluid injection systems.

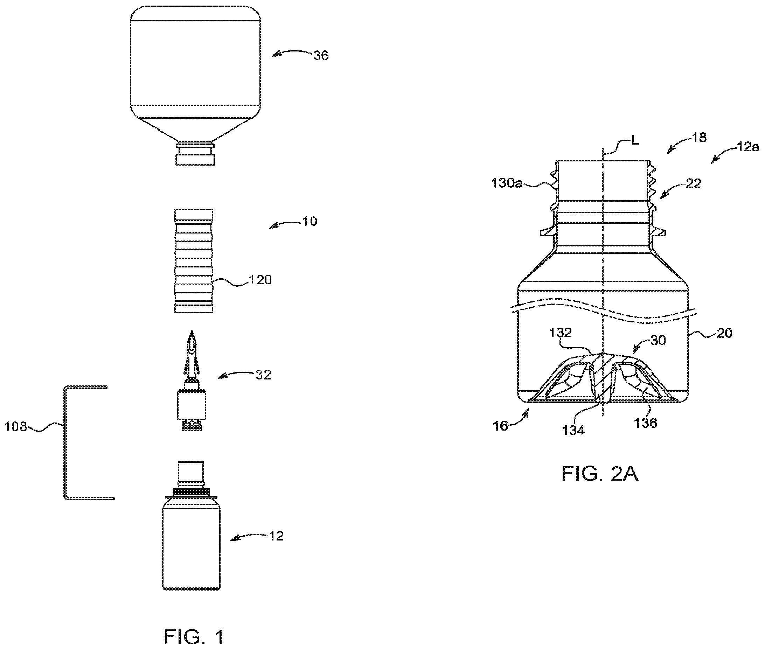

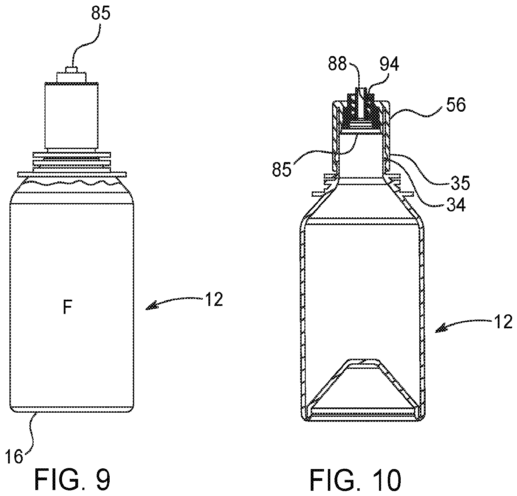

With reference to **FIG. 1** and **FIGS. 3A****-11,** a fluid transfer assembly **10** for delivery of medical liquids is illustrated in accordance with one example. The fluid transfer assembly **10** is configured to facilitate transfer of liquid from a second liquid container **36** to a first liquid container **12.** In some examples, the first liquid container **12** and/or the second liquid container **36** may be a syringe, vial, bottle, bag, or other containment structure configured for receiving a volume of liquid therein. In some examples, the first liquid container **12** may be a syringe, while the second liquid container **36** may be a bulk-liquid storage container, such as a bottle or a bag.

With continued reference to **FIG. 1**, the fluid transfer assembly **10** further includes a fill adapter **32** configured for facilitating a transfer of liquid between the two liquid containers, such as from the second liquid container **36** to the first liquid container **12.** The fill adapter **32** may be removably connectable to the first liquid container **12** and/or the second liquid container **36**. In some examples, the fill adapter **32** may be non-removably connected to one of the first liquid container **12** and the second liquid container **36,** and be removably connectable to the other of the first liquid container **12** and the second liquid container **36.** The fill adapter **32** may have a unitary, single piece structure, or it may be formed from two or more components removably or non-removably coupled together.

The fill adapter **32** may be configured for operation between a first state and a second state. In the first state, liquid flow may be obstructed such that no liquid can be transferred between the two liquid containers, such as from the second liquid container **36** to the first liquid container **12.** In the second state, liquid may flow between the two liquid containers, such as from the second liquid container **36** to the first liquid container **12** through the fill adapter **32.** As described herein, the fill adapter **32** may be movable between a first position and a second position to affect operation between the first and second states, respectively.

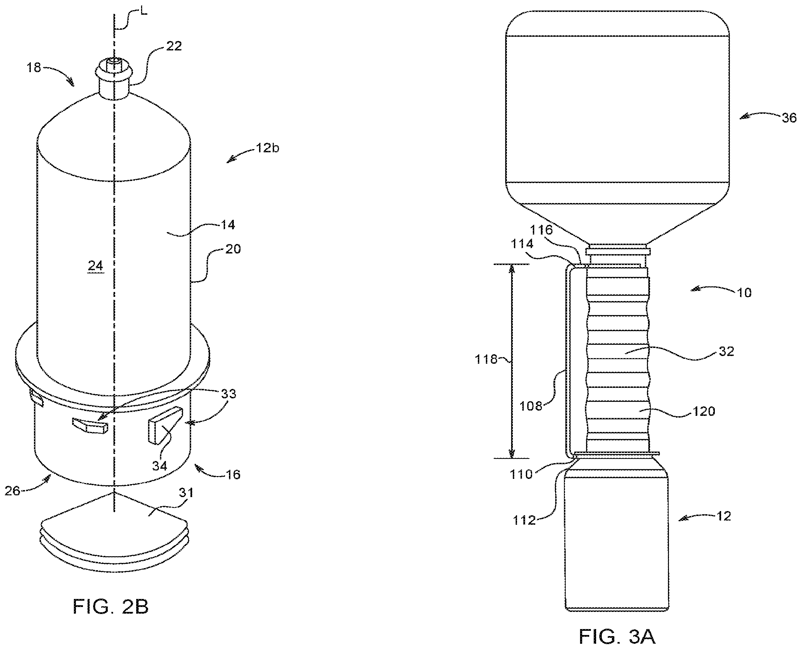

With reference to **FIG. 1** and **FIGS. 3A****-3B**, the fluid transfer assembly **10** may have a locking mechanism **108** removably connectable to at least two of the first liquid container **12**, the fill adapter **32**, and the second liquid container **36.** The locking mechanism **108** may be configured for preventing movement of the components of the fluid transfer assembly **10** between the first position and the second position in order to prevent the flow of liquid between the two containers, such as from the second liquid container **36** to the first liquid container **12.** The locking mechanism **108** may be a bracket that is removable from the fluid transfer assembly **10**, or it may be movable from a first position to a second position. In the first position, the locking mechanism **108** may prevent activation of the fill adapter **32** from the first state to the second state, thereby preventing liquid from flowing from the second liquid container **36** to the first liquid container **12.** In the second position, the locking mechanism **108** may enable the fill adapter **32** to be activated in the second state, thereby allowing liquid to flow from the second liquid container **36** to the first liquid container **12.**

With reference to **FIG. 2A**, a non-limiting example of the first liquid container **12** is shown as a rolling diaphragm syringe **12a** having a flexible sidewall. The rolling diaphragm syringe **12a** may be used as the first liquid container in any example of the present disclosure described with reference to **FIGS. 3A-33C****.** The rolling diaphragm syringe **12a** is adapted for use in CT, MRI, PET, and like procedures and operable at typical operating pressures of, for example, about 10-300 psi, such as 200-300 psi, depending on the viscosity of the liquid and the desired rate of injection. In some examples, the rolling diaphragm syringe **12a** may be configured for use in procedures requiring pressures on the order of 1,200 psi, such as used in angiography. In some aspects, the rolling diaphragm syringe **12a** may be a syringe disclosed in International Patent Application No. PCT/US2015/027582 and/or International Patent Application No. PCT/US2016/028824..

With continued reference to **FIG. 2A**, the rolling diaphragm syringe **12a** generally includes a hollow body that includes a forward or distal end **18**, a rearward or proximal end **16,** and a flexible sidewall **20** extending therebetween along a longitudinal axis **L.** In use, the proximal end **16** is configured for insertion into a throughbore of a pressure jacket attached to a liquid injector such that the sidewall **20** is surrounded by the interior surface of the pressure jacket. At least a portion of the distal end **18** of the rolling diaphragm syringe **12a** may be exposed from the distal end **18** of the pressure jacket. In some examples, the rolling diaphragm syringe **12a** may be formed using a blow-molding technique. In other examples, the rolling diaphragm syringe **12a** may be injection molded.

With continued reference to **FIG. 2A**, the proximal end **16** of the syringe **12a** connects to a closed end wall **30,** and the distal end **18** of the rolling diaphragm syringe **12a** defines a syringe neck **22** opposite the closed end wall **30.** The distal end **18** may have a frusto-conical shape that gradually narrows from the sidewall **20** to the syringe neck **22**. The syringe neck **22** is open to allow liquid to be introduced into and/or delivered from the syringe interior. The closed end wall **30** may be shaped to interface directly or indirectly with a drive member of a liquid injector (not shown). For example, the closed end wall **30** may define a receiving end pocket for interfacing directly with a similarly-shaped drive member, which may be shaped to substantially match the shape of the closed end wall **30.** The sidewall **20** and/or the end wall **30** may have uniform or non-uniform thickness. For example, the sidewall **20** may have increased thickness at the distal end **18** compared to the end wall **30.**

The sidewall **20** of the rolling diaphragm syringe **12a** defines a soft, pliable or flexible, yet self-supporting body that is configured to roll upon itself under the action of the drive member. In particular, the sidewall **20** of the rolling diaphragm syringe **12a** is configured to roll upon itself such that its outer surface is folded and inverted in a radially inward direction as the drive member is moved in a distal direction, and unroll and unfold in the opposite manner in a radially outward direction as the drive member is retracted in a proximal direction.

The rolling diaphragm syringe **12a** may be made of any suitable medical-grade plastic or polymeric material, desirably a clear or substantially translucent plastic material, such as, but not limited to, polypropylene random copolymer, polypropylene impact copolymer, polypropylene homopolymer, polypropylene, polyethylene terephthalate, POM, ABS, HPDE, nylon, cyclic olefin copolymer, multilayer polypropylene, polycarbonate, ethylene vinyl acetate, polyethylene, and the like. The material of the rolling diaphragm syringe **12a** is desirably selected to meet the required tensile and planar stress requirements, water vapor transmission, and chemical/biological compatibility.

The distal end **18** of the rolling diaphragm syringe **12a,** such as the syringe neck **22,** may have a connection member **130a** at the distal end **18** for connecting to a corresponding cap member, for example at least a portion of the fill adapter **32** described herein. In some aspects, the connection member **130a** is a threaded interface having one or more threads for mating with corresponding threads on the fill adapter **32.** In certain aspects, the connection member **130a** may be configured to connect with the fill adapter **32** by way of luer-type connection. In other aspects, the connection member **130a** may have one or more lips or grooves that interact with corresponding grooves or lips on the fill adapter **32** to releasably or non-releasably retain the rolling diaphragm syringe **12a** with the fill adapter **32.** The connection between the fill adapter **32** and the syringe **12a** may have at least one seal, such as an O-ring seal, to prevent liquid leakage at a connection interface between the fill adapter **32** and the syringe **12a.** The syringe neck **22** may also be configured for fluid connection with a liquid path set (not shown) that may be connected to the patient. In some examples, the liquid path set may be in the form of tubing configured for delivering liquid from the first liquid container **12** to the patient or a container for receiving the liquid. In some examples, the liquid path set is removably connectable with the syringe neck **22** of the first liquid container **12.**

The end wall **30** may have a central portion **132** having a substantially dome-shaped structure and a drive member engagement portion **134** extending proximally from the central portion **132,** such as from an approximate midpoint of the central portion **132.** In some aspects, a distal most end of the central portion **132** may be substantially flat. The drive member engagement portion **134** is configured for engagement with the engagement mechanism on the drive member of the liquid injector. The proximal end **16** of the rolling diaphragm syringe **12a** may have one or more ribs **136** protruding radially outward from the drive member engagement portion **134** along a proximal surface of the central portion **132.** In certain embodiments, rolling diaphragm syringe **12a** may be originally in the compressed, rolled configuration when engaged with the injector and the drive member may engage the drive member engagement portion **134** to retract the end wall **30,** unrolling the sidewall **20,** to allow filling of the syringe interior.

With reference to **FIG. 2B****,** a non-limiting example of the first liquid container **12** is shown as a syringe **12b** having a substantially rigid sidewall. The syringe **12b** may be used as the first liquid container in any example of the present disclosure described with reference to **FIGS. 3A-33C****.** The syringe **12b** is adapted for use in CT, MRI, PET, and like procedures and operable at typical operating pressures of, for example, about 10-300 psi, such as 200-300 psi, depending on the viscosity of the liquid and the desired rate of injection. In some examples, the syringe **12b** may be configured for use in procedures requiring pressures on the order of 1,200 psi, such as used in angiography. In some aspects, the syringe **12b** may be a syringe disclosed in U.S. Patent No. 9,173,995.

The syringe **12b** generally has a cylindrical syringe barrel **14** formed from glass, metal, a suitable medical-grade plastic, or a combination thereof. The barrel **14** has a proximal end **16** and a distal end **18,** with a sidewall **20** extending therebetween along a length of a longitudinal axis **L.** A syringe neck **22** extends from the distal end **18** of the barrel **14.** The barrel **14** has an outer surface **24** and an inner surface **26** that defines an interior volume configured for receiving the liquid therein. The proximal end **16** of the barrel **14** may be sealed with a plunger **31** that is slidable through the barrel **14.** The plunger **31** forms a liquid-tight seal against the inner surface **26** of the sidewall **20** of the barrel **14** as it is advanced or retracted therethrough. The plunger **31** may have a rigid inner element configured for engagement with a drive member of a liquid injector (not shown). The plunger **31** may further include an elastomeric cover disposed over at least a portion of the rigid inner element. The elastomeric cover is configured to engage the inner surface **26** of the barrel **14** and provide the liquid-tight seal against the inner surface of the sidewall **20** of the barrel **14.**

With continued reference to **FIG. 2B****,** the proximal end **16** of the syringe **12b** is sized and adapted for being removably inserted in a syringe port of the injector. In some examples, the proximal end **16** of the syringe **12b** defines an insertion section that is configured to be removably inserted into the syringe port of the injector **10** while the remaining portion of the syringe **12b** remains outside of the syringe port.

In certain examples, the proximal end **16** of the syringe **12b** includes one or more syringe retaining members **33** adapted to form a locking engagement with a corresponding locking mechanism in the syringe port of the injector for releasably retaining the syringe **12b** in the syringe port. The combination of the syringe **12b** having the one or more syringe retaining members **33** and the locking mechanism of the injector defines a connection interface for loading and unloading the syringe **12b** to and from the injector. In some examples, at least a portion of the one or more syringe retaining members **33** may cooperate with at least a portion of the locking mechanism to self-orient the syringe **12b** relative to the syringe port such that the syringe **12b** may be releasably inserted into and locked with the syringe port. The one or more syringe retaining members **33** may be formed as one or more lugs **34** that protrude radially outwardly from the outer surface **24** of the syringe barrel **14** relative to the longitudinal axis **L.** In some examples, a plurality of lugs **34** may be separated radially about the circumference of the barrel **14.** In such examples, the lugs **34** are separated from each other by portions of the outer surface **24** of the barrel **14.** Each of the one or more lugs **34** may have a generally triangular, rectangular, polygonal, or arrowhead shape.

With reference to **FIG. 3A****,** the locking mechanism **108** may be a "U"-shaped bracket with a mount **110** at a first end **112** adapted to connect to the first liquid container **12,** and a mount **114** at a second end **116** adapted to connect to the second liquid container **36.** The length of the middle portion **118** of the locking mechanism **108,** defined as the length between the first end **112** and the second end **116,** is longer than a longitudinal length of the fill adapter **32,** so that when the locking mechanism **108** is secured to both the first liquid container **12** and the second liquid container **36** the fill adapter **32** will be in a first engagement position (i.e., engaged with only the first liquid container **12** and not engaged with the second liquid container **36**).

The fluid transfer assembly **10** may further include a flexible shroud **120,** made from any durable and flexible material such as rubber, to enclose and protect the fill adapter **32.** The flexible shroud **120** may be collapsible or compressible to accommodate movement of the fill adapter **32** from the first position to the second position.

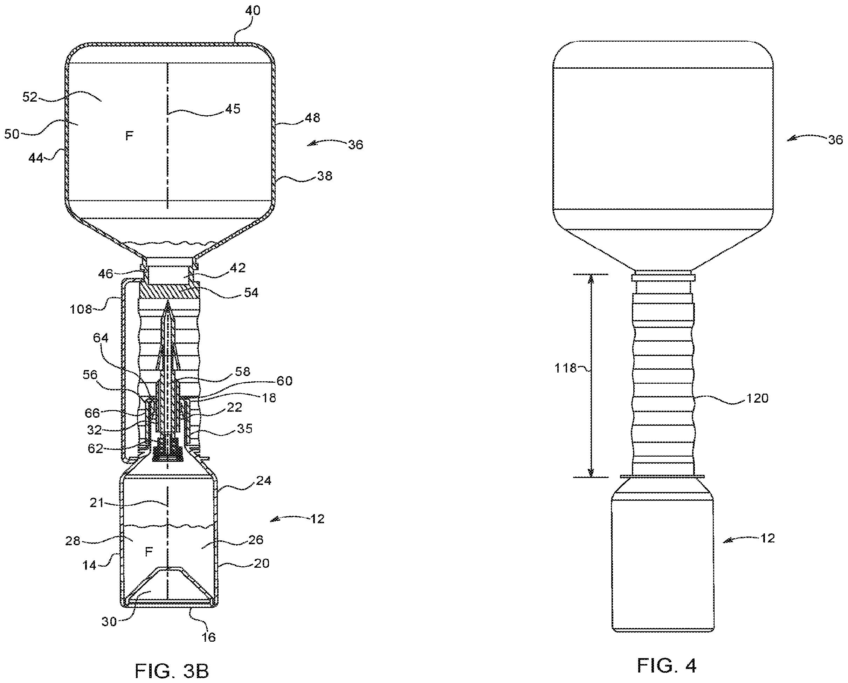

With reference to **FIG. 3B****,** the second liquid container **36** may have a cylindrical barrel **38** formed from glass, metal, a suitable medical-grade plastic, or a combination thereof or may be a plastic bag, such as a saline bag. The barrel **38** has a proximal end **40** and a distal end **42,** with a sidewall **44** extending therebetween along a longitudinal axis **45.** A liquid delivery section, such as a neck **46,** extends from the distal end **42** of the barrel **38.** The barrel **38** has an outer surface **48** and an inner surface **50** that defines an interior volume **52** configured for receiving a liquid **F** therein. The distal end **42** of the second liquid container **36** may include a connection interface for connecting with the fill adapter **32.** In some examples, the distal end **42** may be enclosed by a piercable septum **54.** The fill adapter **32** may be configured to pierce through the septum **54** to connect the interior volume **52** of the second liquid container **36** with the interior volume **28** of the first liquid container **12** to facilitate transfer of liquid **F** from the second liquid container **36** to first liquid container **12.**

With continued reference to **FIG. 3B****,** the fill adapter **32** has a generally cylindrical body **56** formed from glass, metal, a suitable medical-grade plastic, or a combination thereof. The body **56** has a longitudinal axis **58,** a distal end **60,** and a proximal end **62.** The distal end **60** is configured for engaging the second liquid container **36** to establish fluid connection between the second liquid container **36** and the fill adapter **32.** The proximal end **62** is configured for engaging the first liquid container **12** to establish fluid connection between the first liquid container **12** and the fill adapter **32.** In some examples, the proximal end **62** of the body **56** is configured to releasably connect to the syringe neck **22.**

The body **56** of the fill adapter **32** is desirably hollow to allow a passage of liquid therethrough. In some examples, the body **56** has an inner sidewall **64** and an outer sidewall **66** extending between the distal end **60** and the proximal end **62** along the longitudinal axis **58.** In some examples, the inner sidewall **64** may be configured to interface with the inner surface **26** of the syringe neck **22** while the outer sidewall **66** is configured to interface with the outer surface **24** of the syringe neck **22.** The groove **35,** configured to engage the tabbed portion **34** located on the outer surface **24** of syringe neck **22,** is formed on outer sidewall **64.**

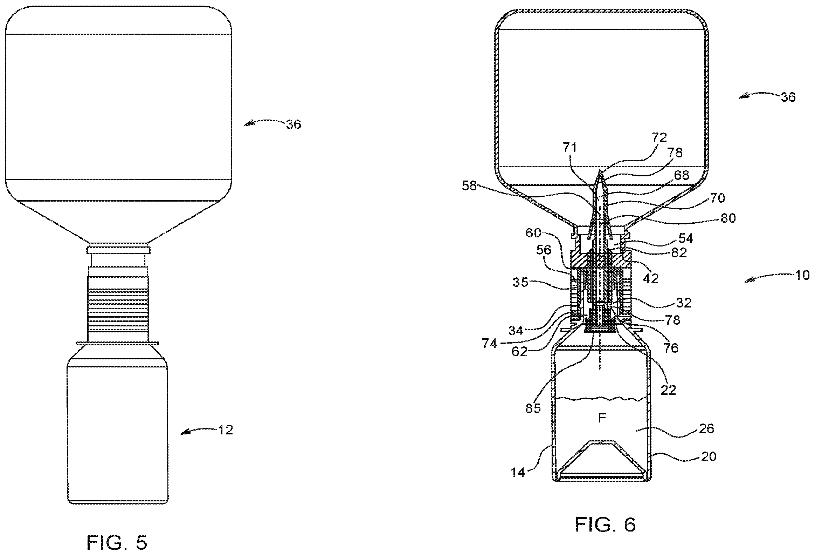

With specific reference to **FIG. 6****,** the distal end **18** of the first liquid container **12** may include a connection interface for connecting the first liquid container **12** with a fill adapter **32.** In some examples, the connection interface may include a tabbed portion **34** located on the outer surface **24** of the barrel **14** on the syringe neck **22** to engage with the groove **35** of the fill adapter **32** to secure the fill adapter **32** to the first liquid container **12.** When engaged, the fill adapter **32** facilitates the transfer of liquid **F** from a second liquid container **36** to the first liquid container **12.** In other examples, the connection interface between the first liquid container **12** and the fill adapter **32** may be a threaded connection, a welded connection, a molded connection, an interference fit connection, a snap fit connection, or other mechanical connection.

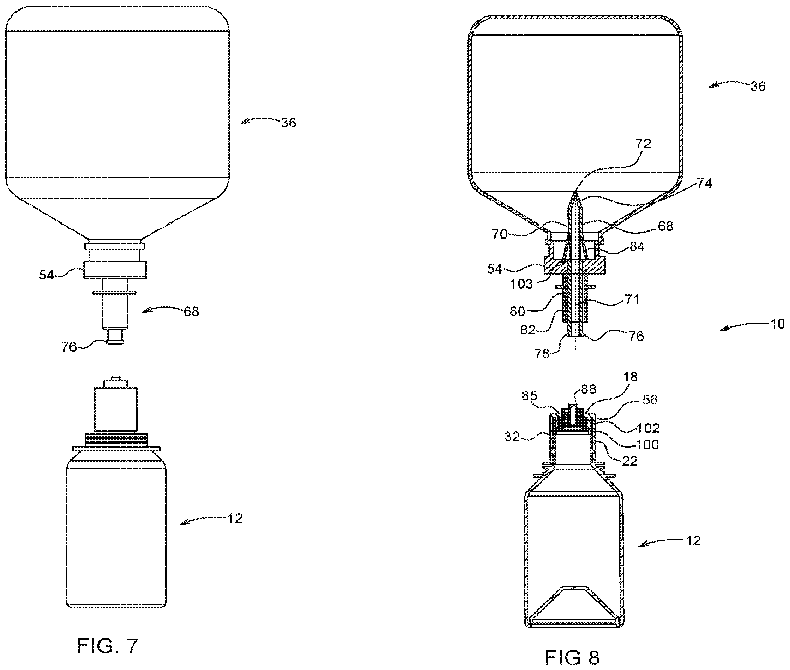

With specific reference to **FIG. 8****,** the body **56** removably receives a piercing device **68** configured for penetrating the piercable septum **54** of the second liquid container **36.** The piercing device **68** has a generally cylindrical body **70** and includes a longitudinal axis **71,** a piercing point **72** located on a distal end **74,** and a flared base **76,** flaring radially away from the longitudinal axis **71,** located on a proximal end **78.** In some examples, the flared base **76** is shaped to promote fluid flow due to the Coand 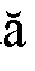 effect. With reference to **FIG. 6****,** when received within the body **56** of the fill adapter **32,** the body **56** of the fill adapter **32** surrounds the body **70** of the piercing device **68** and the piercing point **72** may extend beyond the distal end **60,** while the flared base **76** extends toward the proximal end **62** and into the syringe neck **22.** The longitudinal axis **71** of the piercing device **68** may be coaxial with the longitudinal axis **58** of the body **56.** The piercing device **68** has a first conduit **80** surrounded by a second annular conduit **82.** The first conduit **80** is configured to displace air from the interior of the first liquid container **12** during a filling procedure, while the second annular conduit **82** is configured to transfer liquid **F** from the second liquid container **36** to the first liquid container **12.** With some examples, a cross-sectional area of the first conduit **80** can be between 8% and 40% smaller than a cross-sectional area of the second annular conduit **82.** Both the first conduit **80** and the second annular conduit **82** extend along the longitudinal axis **71** of the piercing device **68.** The first conduit **80** and the second annular conduit **82** may be of equal or different lengths. The first conduit **80** may be concentric with the second annular conduit **82.** In some examples, the first conduit **80** and the second conduit **82** may be parallel to each other in a side-by-side arrangement. The first conduit **80** and/or second annular conduit **82** may have a smooth or helical inner sidewall (not shown). With some examples, a ratio of a cross-sectional area of the flared base **76** to a cross-sectional area of the first conduit **80** may be between 5:1 to 20:1. With further examples, a radius of the flared base **76** is desirably less than or equal to a radius of the syringe **12** at a transition between the frusto-conical distal end and the syringe neck **22.**

With reference to **FIG. 6****,** the fluid transfer assembly **10** is shown in a second engagement position, where the piercing point **72** penetrates the rubber septum **54** of the second liquid container **36,** thereby permitting liquid **F** to enter the second annular conduit **82.** Liquid **F** travels from the proximal end **78** of the second annular conduit **82** through the fill adapter **32** and into the interior volume **28** of the first liquid container **12** via the syringe neck **22.** Liquid flow may be driven by gravity, or it may be vacuum-assisted, such as when the rolling diaphragm syringe **12a** is unrolled in the proximal direction, or when the plunger **31** of the syringe **12b** is withdrawn in the proximal direction. The liquid **F** entering the first liquid container **12** via the second annular conduit **82** contacts the flared base **76** and is deflected radially outward from the longitudinal axis **71** and towards the inner surface **26** of the barrel **14** before dripping down the sidewall **20** of the barrel **14** and accumulating at the bottom of the first liquid container **12.** The liquid **F** initiates flow through the second annular conduit **82** instead of the first conduit **80** because it is at a lower point of the fill adapter **32** and will have a higher head pressure than the inlet for the first conduit **80.** The entry of liquid **F** from the second liquid container **36** into the interior of the first liquid container **12** displaces air contained inside the first liquid container **12** into the second liquid container **36** through the first conduit **80.** Any liquid initially flowing through the first conduit **80** is forced out from the first conduit **80** by the air flowing through the first conduit **80** from the first liquid container **12** into the second liquid container **36.** Air/liquid exchange within the first fluid container **12** occurs substantially simultaneously through the first conduit **80** and the second annular conduit **82,** without any flow hesitation or gurgling due to uneven flow characteristics.

With reference to **FIG. 8****,** the piercing device **68** may further include a plurality of deflectable barbs **84,** configured to prevent removal of the piercing device **68** from the second liquid container **36** after the piercing device **68** penetrates the septum **54.** The deflectable barbs **84** extend radially outward from the outer surface of the piercing device **68** and form an acute angle therewith. As the piercing point **72** penetrates the piercable septum **54** of the second liquid container **36,** the barbs **84** deflect radially inwards towards the longitudinal axis **71** so as to permit the piercing device **68** to pass through the septum **54** and into the second liquid container **36.** Likewise, upon exerting a proximally directed force on the piercing device **68,** the deflectable barbs **84** extend radially outward to prevent the removal of the piercing device **68** from the second liquid container **36.** The proximally directed force causes the piercing device **68** to be disconnected from the body **56** of the fill adapter **32,** while the body **56** remains connected to the syringe neck **22** of the first liquid container **12.** In this manner, the second liquid container **36** can be discarded, along with the piercing device **68** trapped within the syringe neck **46** of the second liquid container **36.**

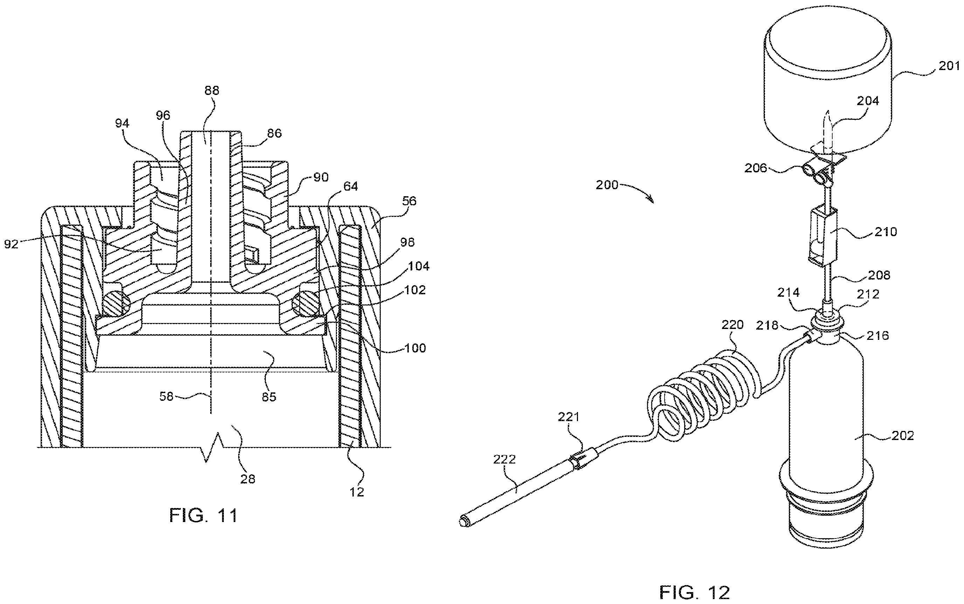

With reference to **FIG. 8****,** the fill adapter **32** may further include a plug **85** that is releasably connected to the distal end **78** of the piercing device **68.** With the removal of the piercing device **68** from the body **56,** the plug **85** is moved from a first position (shown in **FIG. 6**) to a second position (shown in **FIG. 8**). In the first position, the plug **85** is disconnected from the body **56,** while in the second position the plug **85** is connected to the body **56** as described herein. With specific reference to **FIG. 11****,** the plug **85** has an inner member **86** defining a liquid channel **88** that is in liquid communication with the interior volume **28** of the first liquid container **12.** The inner member **86** is in alignment with the first conduit of the piercing device (not shown in **FIG. 11**). The inner member **86** is surrounded by an outer annular skirt **90** that extends axially along at least a portion of the longitudinal length of the inner member **86.** The inner member **86** is spaced apart radially from the outer annular skirt **90** by an annular space **92.** The outer annular skirt **90** has a threaded portion **94,** i.e., a female luer lock member formed on an inner surface **96** facing toward the inner member **86,** configured for connecting the first liquid container **12** to a liquid path. An outer surface **98** of the outer annular skirt **90** has at least one foot **100** extending from a portion of a longitudinal length of the outer annular skirt **90** adapted to fit within a correspondingly shaped recess **102** formed on the inner sidewall **64** of the body **56.** Once the foot **100** is engaged within the recess **102,** the plug **85** is connected to the body **56** to prevent the plug **85** from being removed from the tip of the syringe neck **22.** With specific reference to **FIG. 8****,** when the foot **100** is engaged within the recess **102** and the user exerts a proximally directed force on the first liquid container **12** to separate the assembly **10,** a bottom portion **103** of the barbs **84** contact the septum **54** of the second liquid container **36** and prevent the piercing device **68** from being withdrawn from the second liquid container **36.** As a result, the body **70** of the piercing device **68** and the plug **85** attached to the distal end **78** of the piercing device **68** is forced towards the distal end **18** of the first liquid container **12** until the foot **100** locks within the recess **102.** Further, proximal force causes the body **70** of the piercing device **68** to detach from the body **56** of the fill adapter **32,** while the plug **85** remains connected to the body **56** and covers the syringe neck **22** of the first liquid container **12.** The piercing device **68** may be discarded along with the second liquid container **36.**

With reference to **FIG. 11****,** tubing (not shown) may optionally be connected to the plug **85,** establishing a liquid path from the first liquid container **12** to a patient after the first liquid container **12** has been filled with liquid. In some examples the outer annular skirt **90** of the plug **85** contains a threaded portion **94,** i.e., a female luer lock member, formed on an inner surface **96** configured to connect with tubing having a male member (not shown) to create a liquid path. In other examples, the arrangement of the female threading **94** and the male member (not shown) may be reversed. In such examples, the male element is provided on the plug **85,** while the tapered female threading **94** is provided on the tubing.

With continued reference to **FIG. 11****,** the inner sidewall **64** of the body **56** may radially flex the at least one foot **100** towards the longitudinal axis **58,** to facilitate movement of the plug **85** in a longitudinal direction within the syringe neck **22** when the foot **100** is not engaged within the recess. The outer surface **98** of the plug **85** may further include one or more seals **104** to prevent liquid **F** from leaking between the body **56** and the plug **85** when the foot **100** is engaged within the recess **102.** Alternatively, the one or more seals **104** may optionally be secured on the inner sidewall **64** around the recess **102.**

Next, referring to **FIG. 12****,** a fluid transfer assembly **200** in accordance with another example of the present disclosure is discussed. The fluid transfer assembly **200** is configured to eliminate the need for vacuum to fill the syringe through activation of the plunger. Instead, the fluid transfer assembly **200** relies upon head pressure of the liquid to increase the fill rate by maintaining the liquid container at a height above the syringe during the filling process. As shown in **FIG. 12****,** the fluid transfer assembly **200** has a syringe **202** and a spike adapter **204** configured for connection to a liquid container **201** above syringe **202**. The spike adapter **204** comprises a vent **206** to allow air into the liquid container during the filling process. Connecting the syringe **202** and the spike adapter **204** is a fill tube **208**, with the fill tube **208** having a tube clamp **210** placed thereon for selectively stopping or starting the flow of liquid through the fill tube **208**. The tube clamp **210** may be any appropriate alternative valve, such as a pinch valve. While the syringe **202** may be connected directly to the spike adapter **204,** the fill tube **208** increases the head height of the liquid in the liquid container **201** by increasing the distance between the syringe **202** and the liquid container **201.** In this manner, the fill rate of the syringe **202** can be increased because flow rate generally increases with head height when the remaining flow variables are kept constant. In various embodiments, the fill rate for syringe **202** may be selected by selecting a corresponding head height.

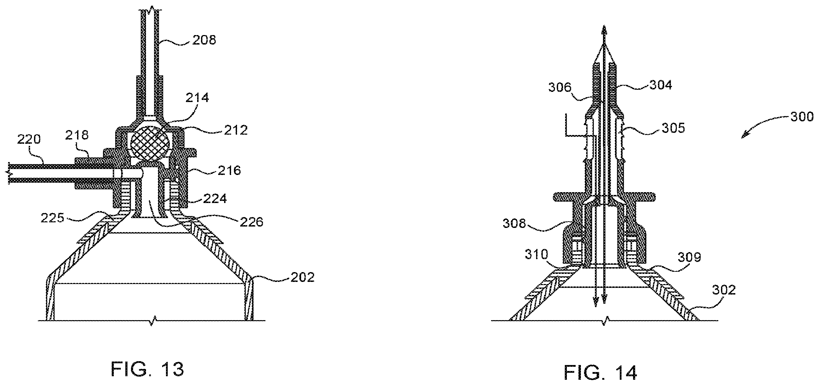

With reference to **FIG. 13****,** and with continued reference to **FIG. 12****,** a valve housing **216** is coupled to the open end of the syringe **202**, with the valve housing **216** having a connecting cap **212** thereon and an optional floating ball **214** or other valve design allowing one-way fluid flow retained therein. The valve housing **216** further has a fitting **218,** configured for connection with a low-pressure connector tube **220**, which may form a wet connection with a separate tube (not shown) connected to the patient once the syringe is filled and the tubing primed. The connector tube **220** has a prime straw **222** connected to a fitting **221** at a distal end thereof. In examples where the floating ball **214** is omitted, a separate valve mechanism (not shown) may be provided to prevent liquid from flowing from the syringe **202** to the liquid container **201.**

With continued reference to **FIG. 13****,** the valve housing **216** is shown coupled to the syringe **202,** with the fill tube **208** liquidly coupled to the valve housing **216** via the connecting cap **212.** Also within the valve housing **216** is a bell-shaped flow controller **224.** An open end **226** is configured for fluid communication with the connector tube **220**. During the filling process, a liquid from the liquid container **201** (shown in **FIG. 12**) above the syringe **202** is gravity-fed through the fill tube **208** to the valve housing **216.** The floating ball **214** or other one-way valve is configured to allow liquid to flow through the valve housing **216** during the initial fill process, with the liquid flowing down and around an exterior surface of the flow controller **224.** Optionally, the bell-shaped contour of the surface of the flow controller **224** urges the liquid along the interior walls of syringe **202** by a ribbed connector **225.** The combination of the bell-shaped contour of the flow controller **224** and the conical sidewall of the distal end of the syringe **202** (and/or the ribbed connector **225**) results in the liquid filling the syringe **202** in accordance with the Coand 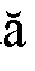 effect. As used herein, the Coand 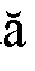 effect is the tendency for a liquid stream to be attracted to a nearby curved or angled surface as the liquid flows along the surface. Thus, as liquid flows down the flow controller **224** and/or the ribbed connector **225,** it is naturally attracted to the inside surface of the conical distal end **250** of the syringe **202**, rather than dripping from the edge of the flow controller **224** and/or the ribbed connector **225.** The liquid then flows down the tubular sidewall **252** of the syringe **202**, ultimately accumulating at the bottom of the syringe **202**, filling syringe **202** from the bottom up as air escapes the syringe through flow controller **224** and connector tube **220.** This flow along the inside surface of the syringe **202** helps to reduce turbulence as the liquid fills the syringe **202**, which aides in reducing air bubbles from forming as the syringe **202** is filled.

As the syringe **202** is filled, the liquid will eventually reach a level within the syringe **202** where it will flow into the open end **226** of the flow controller **224** and flow into the connector tube **220**, thereby priming the connector tube **220.** When the connector tube **220** is fully primed, any additional liquid flow into the syringe **202** will force the floating ball **214** upward against inner surfaces of the connector cap **212,** thereby stopping the flow of liquid from the fill tube **208** into the syringe **202.** At this point, the prime straw **222** may be removed and fitting **221** of the connector tube **220** may be connected to a patient-side tube (not shown), and the contents of the syringe **202** may be delivered the patient via a conventional liquid delivery procedure, such as using a powered liquid injector. During an injection procedure where the liquid is delivered from the syringe **202,** the floating ball **214** seats against the distal end of the valve housing **216** to prevent the flow of liquid into the liquid container **201.** It is to be understood that the rate of liquid flow in the fluid transfer assembly **200** may be optimized via, for example, changes in height between the container and the syringe **202** to increase head pressure, removal of liquid restrictions in the valve areas, increase in the syringe neck size, etc.

Next, referring to **FIG. 14****,** a fill adapter **300** in accordance with another alternative example of the present disclosure is shown. The fill adapter **300** can be used in combination with a syringe **302** having a spike **304** coupled thereto via a snap-on connection, a threaded connection, welding, etc. The spike **304** has a plurality of liquid openings **305** on side surfaces thereof, along with an air exchange conduit **306** running along an axial length therethrough. With some examples, a cross-sectional area of the air exchange conduit **306** can be between 8% and 40% smaller than a cross-sectional area of the conduit in fluid communication with the liquid openings **305.** Liquid pressure at the opening **305** is higher than at the air exchange conduit **306** due to head pressure. Thus, liquid in the container will naturally flow through the opening **305.** Such an arrangement assures that air exiting from the conduit **306** will not enter into the openings **305** to introduce air bubbles into the syringe during a filling procedure. At an end of the air exchange conduit **306** proximal to the syringe **302** is a bell-shaped flow controller **308.** While not shown, the spike **304** is configured for connection to a container (such as the liquid container **201** shown in **FIG. 12**) above the syringe **302** so as to initiate a flow of liquid from the container into the syringe **302.** The liquid within the container is configured to flow into the liquid openings **305** on the spike **304,** flowing downward through spike **304** around the periphery of the air exchange conduit **306.** Air present within the syringe **302** is simultaneously exchanged with air within the container via the air exchange conduit **306.** As the liquid flows along the outer peripheral surfaces of the flow controller **308,** it reaches a bell-shaped flare **310** on a proximal end of the flow controller **308,** urging the liquid toward a ribbed connector **309** at a distal end of the syringe **302.** As discussed above with respect to **FIG. 13****,** this combination of the bell-shaped contour of the flow controller **308** and the ribbed connector **309** results in the liquid filling the syringe **302** in accordance with the Coand 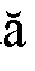 effect. Thus, as liquid flows down the flow controller **308** and the ribbed connector **309,** it is naturally attracted to the inside surface of the syringe **302,** helping to reduce turbulence as the liquid fills the syringe **302** and aiding in the reduction of air bubbles within the syringe **302.** With some examples, a ratio of a cross-sectional area of the bell-shaped flare **310** to a cross-sectional area of an internal conduit of the flow controller **308** may be between 5:1 to 20:1. With further examples, a radius of bell-shaped flare **310** is desirably less than or equal to a radius of the sidewall of the syringe **302** at a transition between the frusto-conical distal end and the syringe neck.

After the syringe **302** is filled with liquid, the spike **304** may be removed from the container and a connector tube (not shown) may be attached thereto via an appropriate connection, such as a snap-on connection. The connector tube may be connected to a patient-side tube (not shown), and the contents of the syringe **302** may be delivered to the patient via a liquid delivery procedure, such as using a powered liquid injector.

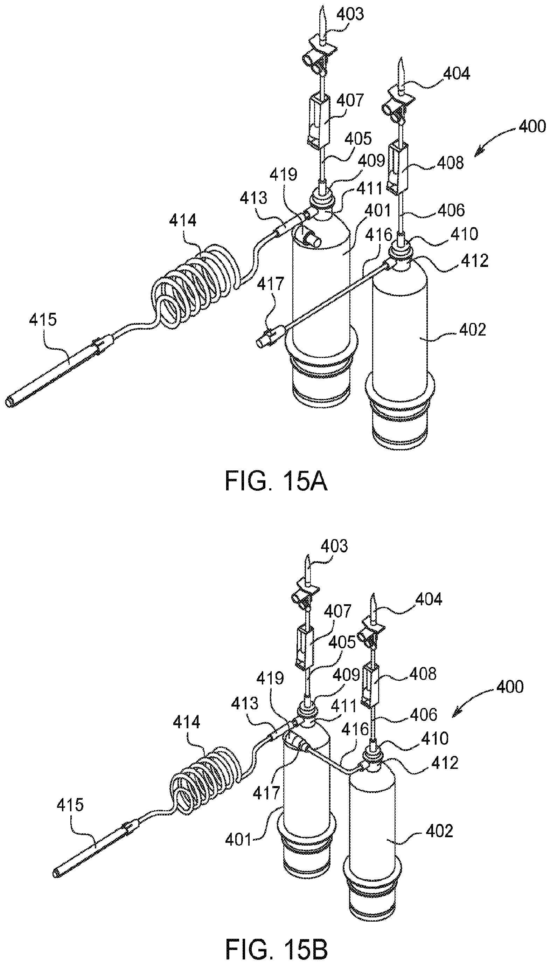

Referring to **FIGS. 15A-15B****,** a fluid transfer assembly **400** in accordance with another example of the present disclosure is shown. The fluid transfer assembly **400** has dual syringes **401, 402.** As is known in the art, many injection procedures involve the injection of two distinct liquids into the patient, for example a contrast agent and saline. The fluid transfer assembly **400** accommodates such a procedure through the use of dual syringes **401, 402.** Each syringe **401, 402** is filled in a substantially similar fashion as that described herein, for example as described according to **FIGS. 12-14****.** That is, the syringes **401, 402** and spike adapters **403, 404** are configured for connection to a liquid container (not shown) above the syringes **401, 402.** Connecting the syringes **401, 402** and the respective spike adapters **403, 404** are fill tubes **405, 406,** with each fill tube **405, 406** having a tube clamp **407, 408** placed thereon for selectively stopping or starting the flow of liquid through the fill tubes **405, 406,** respectively.

Each syringe **401, 402** is coupled to a respective valve housing **411, 412** having respective connector caps **409, 410.** Accordingly, syringes **401, 402** are filled with respective liquids in substantially the same manner as that described above with respect to **FIGS. 12-14****.** As shown in **FIG. 15A****,** the valve housing **411** is liquidly coupled to a T-connector **413.** The T-connector **413** couples the valve housing **411** to a low-pressure connector tube **414,** with the connector tube **414** having a prime straw **415** connected at a distal end thereof. The T-connector **413** also comprises a fitting **419.** On the other hand, valve housing **412** on syringe **402** is coupled directly to a connector tube **416** having a fitting **417** on a distal end thereof.

After filling of respective syringes **401, 402** in a manner similar to that described above with respect to **FIGS. 12-14****,** the fluid transfer assembly **400** provides that the contents of the syringes **401, 402** may be simultaneously or sequentially injected into the patient through connector tube **414.** That is, referring to **FIG. 15B****,** after filling of the syringes **401, 402,** the connector tube **416** and the fitting **417** may be coupled to the fitting **419** on the T-connector **413.** With this fluid connection, the respective liquids within the syringes **401, 402** may be combined together or flowed sequentially during the injection process and provided to the patient via a wet connection to the connector tube **414.**

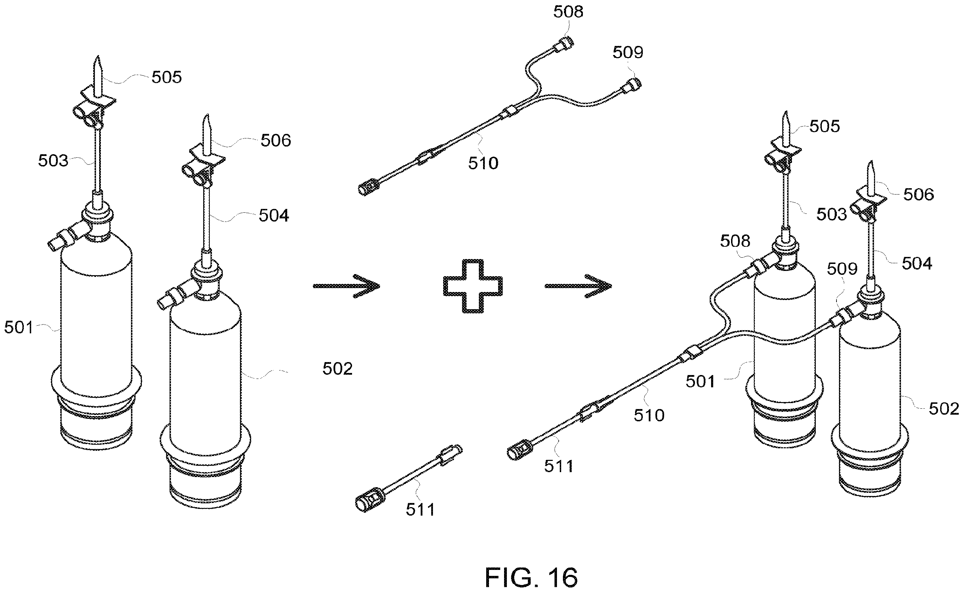

**FIG. 16** illustrates another alternative example of the present disclosure. Dual syringes **501, 502** are shown as liquidly coupled to respective spike adapters **505, 506** via respective fill tubes **503, 504.** The filling procedure may be substantially similar to that described above with respect to **FIGS. 12-13** and **FIGS. 15A-15B****.** After the syringes **501, 502** are filled with the appropriate liquids, they may be coupled to a Y-shaped connector tube **510** via respective check valve fittings **508, 509.** The connector tube **510** may then be connected to a patient-side tube **511** at a distal end thereof. With this fluid connection, the respective liquids within the syringes **501, 502** may be combined together or delivered sequentially during the injection process and provided to the patient via a wet connection to the connector tube **510.** Such a configuration may be particularly effective for multi-patient injector applications, as the connector tube **510** can be a disposable connection and the check valve fittings **508, 509** may prevent bi-directional flow of liquids and contamination of the respective syringes **501, 502.**

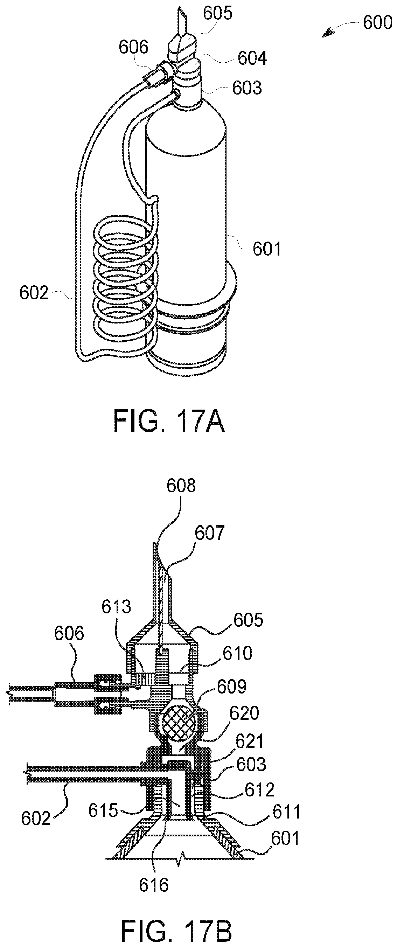

Referring now to **FIGS. 17A-17B****,** a fluid transfer assembly **600** in accordance with another example of the present disclosure is described. **FIG. 17A** illustrates a syringe **601** having a valve housing **603** coupled thereto, wherein the valve housing **603** has a connector cap **604** connected thereon. A spike adapter **605** is coupled to the connector cap **604,** while a low-pressure connector tube **602** is liquidly connected between the valve housing **603** and the connector cap **604,** with a fitting **606** configured to provide the removable connection between the connector tube **602** and the connector cap **604.** **FIG. 17B** provides greater detail of the liquid paths associated with fluid transfer assembly **600.** For example, the spike adapter **605** has a pair of conduits **607, 608.** The liquid conduit **607** allows liquid from within a container (not shown) coupled to the spike adapter **605** to flow down to a conduit **610** within the connector cap **604.** Air conduit **608** allows air from the container to escape as the fluid is introduced through the liquid conduit **607.** With some examples, a cross-sectional area of the air conduit **608** can be between 8% and 40% smaller than a cross-sectional area of the liquid conduit **607.** As shown in **FIG. 17B****,** the distal end of air conduit **608** is positioned distally from the distal end of liquid conduit **607.** With some examples, the air conduit **608** may extend 0.15 in. to 0.25 in. distally further than the liquid conduit **607.** In this manner, liquid pressure at the opening of the liquid conduit **607** is higher than liquid pressure at the air conduit **608** due to difference in head pressure. Due to this difference in pressure, air can be forced out of the container through the air conduit **607** as the container is being filled through the liquid conduit **608.** In addition, introduction of fluid into the first fluid container through the liquid conduit **608** also causes a vacuum in the second fluid container, which "pulls" the air from the first fluid container into the second fluid container through the air conduit **608.** As the syringe **601** is being filled, liquid is able to flow around an optional floating ball **609** within the valve housing **603** and through a passage **620.** The liquid is then able to flow around the periphery of a bell-shaped flow controller **612** via a flow passage **621.** As discussed above with respect to **FIGS. 12** and **13****,** the flow controller **612** is shaped and sized so as to direct the liquid to flow along the inner surfaces of syringe **601** as syringe **601** is filled via the Coand 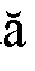 effect. A flared portion **616** of the flow controller **612** and the ribbed connector **611** on a distal end of syringe **601** may also direct such liquid flow. With some examples, a ratio of a cross-sectional area of the flared portion **616** to a cross-sectional area of the flow passage **621** may be between 5:1 to 20:1. With further examples, a radius of the flared portion **616** is desirably less than or equal to a radius of the sidewall of the syringe **601** at a transition between the frusto-conical distal end and the syringe neck.

As the liquid from the container fills syringe **601,** air within the syringe **601** is able to pass through a passage **615** formed in the flow controller **612,** with the air then passing through the connector tube **602** and into a conduit **608** of the spike adapter **605,** where the air enters the container. As the syringe **601** fills with liquid, the liquid itself enters the passage **615** and primes the connector tube **602.** The liquid is prevented from entering the conduit **608** (and thus re-entering the container) via a filter **613** at which point connector tube **602** is primed with liquid. After the connector tube **602** is fully primed with liquid, the liquid still entering the syringe **601** urges the floating ball **609** upward, effectively sealing the syringe **601** from receiving more liquid. During an injection procedure where the liquid is delivered from the syringe **601,** the floating ball **609** seats against the distal end of the spike adapter **605** to prevent the flow of liquid into the liquid container (not shown).

After the syringe **601** is filled with contrast or saline and the connector tube **602** is fully primed, the connector tube **602** may be detached from the connector cap **604** at the fitting **606,** and a patient-side connector tube can, in turn, be connected to the fitting **606** such that the contents of the syringe **601** may be delivered to the patient via a conventional liquid delivery procedure, such as using a powered liquid injector.

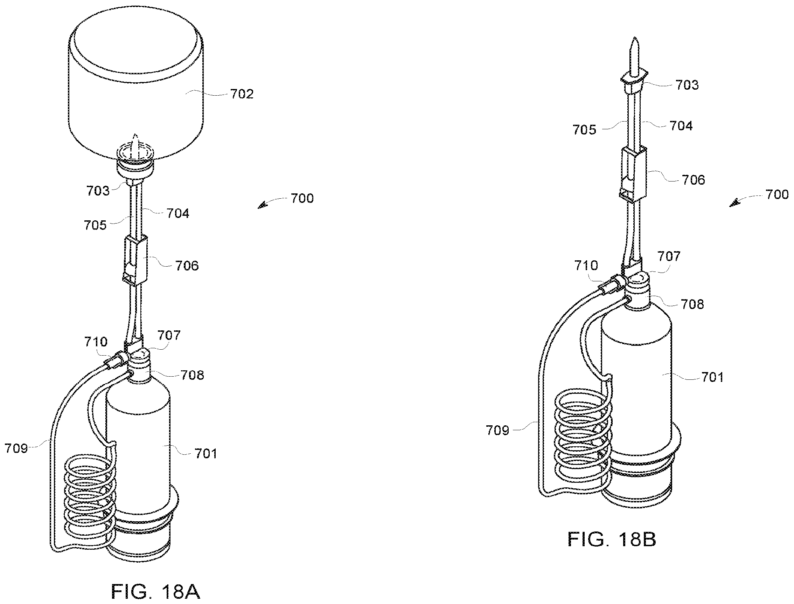

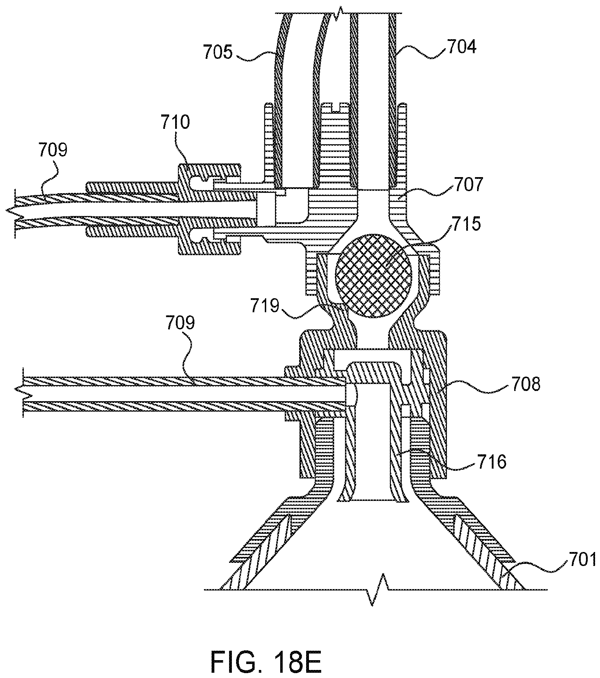

Referring to **FIGS. 18A-18E****,** a fluid transfer assembly **700** in accordance with another example of the present disclosure is described. Many of the components of system **700** are the same or similar to those of system **600** described above with respect to **FIGS. 17A-17B****,** so duplicative components will not be discussed in detail. The fluid transfer assembly **700** has a syringe **701** liquidly coupled to a container **702** via two distinct connector tubes **704, 705.** One end of the respective connector tubes **704, 705** is coupled to a spike adapter **703** that provides an engagement with the container **702.** The syringe **701** has a valve housing **708** coupled thereto, wherein the valve housing **708** has a connector cap **707** connected thereon. Respective connector tubes **704, 705** are coupled to the connector cap **707,** while a low-pressure connector tube **709** is liquidly connected between the valve housing **708** and the connector cap **707,** with a fitting **710** configured to provide the removable connection between the connector tube **709** and the connector cap **707.** Additionally, a tube clamp **706** (or other appropriate valve) is coupled to the connector tube **705** to enable the flow through the connector tube **705** to be shut off.

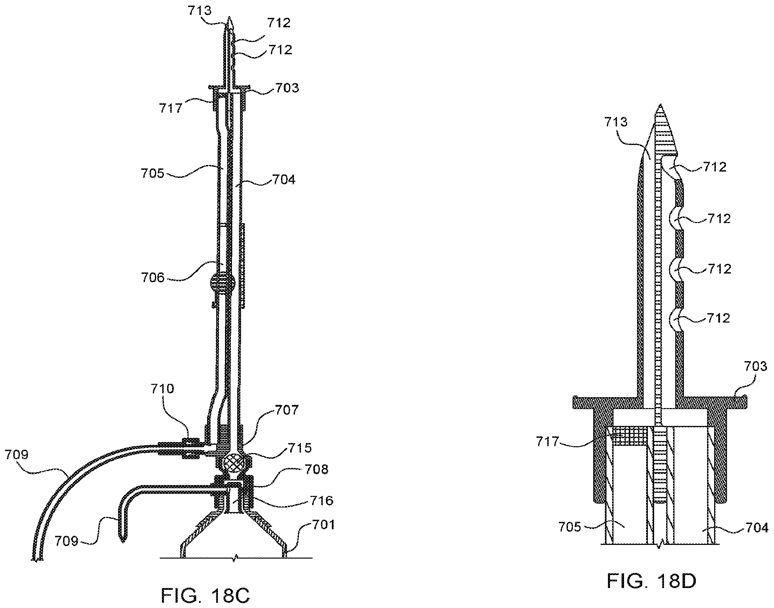

Referring specifically to **FIGS. 18C-18E****,** greater detail of the liquid paths associated with the fluid transfer assembly **700** is shown. For example, the spike adapter **703** has a plurality of liquid openings **712** extending laterally through the spike adapter **703** configured to enable liquid from the container **702** to enter the connector tube **704.** The spike adapter **703** also has a conduit **713** configured for air communication with the connector tube **705** and the headspace of container **702.** Openings **712** allow liquid from the container **702** to flow down to the connector cap **707.** The openings **712** may have a cross-sectional area of from 0.01 to 0.10 in² or around 0.06 in². With some examples, a cross-sectional area of the conduit **713** can be between 8% and 40% smaller than a cross-sectional area of the conduit in fluid communication with the openings **712.** As the syringe **701** is being filled, liquid is able to flow around an optional floating ball **715** within the valve housing **708** and into the syringe **701,** as the floating ball **715** is held on a surface **719** within the valve housing **708** that enables liquid flow thereby. Once again, as discussed above with respect to **FIGS. 12-13****,** a flow controller **716** is shaped and sized so as to direct the liquid to flow along the inner surfaces of the syringe **701** as the syringe **701** is filled via the Coand 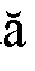 effect.

As the liquid from the container **702** fills the syringe **701,** air within the syringe **701** is able to pass through the flow controller **716,** with the air then passing through a connector tube **709** and into the conduit **713** of the spike adapter **703,** where the air enters the container **702** (shown in **FIG. 18A**). As the syringe **701** fills with liquid, the liquid itself enters the flow controller **716** and primes the connector tube **709.** The liquid is prevented from entering the conduit **713** (and thus re-entering the container) via a filter **717.** After the connector tube **709** is fully primed with liquid, the liquid still entering the syringe **701** urges the floating ball **715** upward, effectively sealing the syringe **701** from receiving more liquid. During an injection procedure where the liquid is delivered under pressure from the syringe **701,** the floating ball **715** seats against the distal end of the valve housing **708** to prevent the flow of liquid into the liquid container **702.** Flow through tubes **703,704** may also be prevented by tube claim **706.**

With specific reference to **FIG. 18D****,** the openings **712** may be spaced apart axially relative to one another. When the spike adapter **703** is inserted into the liquid container, liquid pressure at the openings **712** from the liquid in the container increases from the distal end of the spike adapter **703** toward the proximal end of the spike adapter **703.** In this manner, the liquid pressure at the distal most opening **712** will be slightly lower than the liquid pressure at the proximal most opening **712.** Thus, liquid in the container will first flow through the proximal most opening **712** before flowing through the distal openings **712.** Such an arrangement assures that air exiting from the conduit **713** will not enter into the openings **712** to introduce air bubbles into the syringe during a filling procedure, for example due to a Venturi effect.

After the syringe **701** is filled and the connector tube **709** is fully primed, the connector tube **709** may be detached from the connector cap **707** at the fitting **710,** and a patient-side connector tube can, in turn, be connected to the fitting **710** such that the contents of the syringe **701** may be delivered to the patient via a conventional liquid delivery procedure, such as using a powered liquid injector.

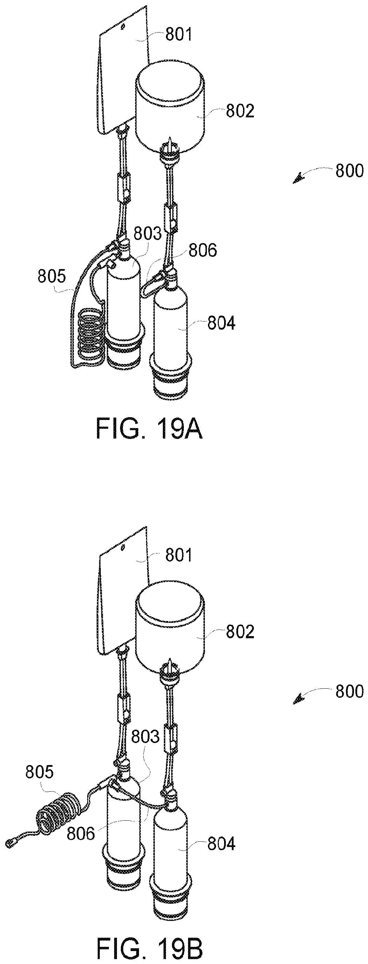

Turning to **FIGS. 19A-19B****,** a fluid transfer assembly **800** in accordance with another example of the present disclosure is shown. The fluid transfer assembly **800** has a first container (e.g., a saline bag) **801** and a second container **802** (e.g., a bottle of contrast agent) liquidly coupled to respective first and second syringes **803, 804.** The details of how the first and second syringes **803, 804** are filled is substantially similar to that disclosed above with respect to **FIGS. 18A-18E****,** so further description of the fill process will be omitted herein. As **FIG. 19A** shows, during the fill process, a low-pressure connector tube **805** forms a passageway between the first syringe **803** and a connector tube leading to the first container **801,** while a separate tube **806** provides the passageway between the second syringe **804** and a connector tube leading to the second container **802.** However, referring to **FIG. 19B****,** after filling of the first and second syringes **803, 804** with the respective liquids, the connector tube **806** may be coupled to the connector tube **805.** With this fluid connection, the respective liquids within the first and second syringes **803, 804** may be combined together or delivered sequentially during the injection process and provided to the patient via a wet connection to the connector tube **805.**

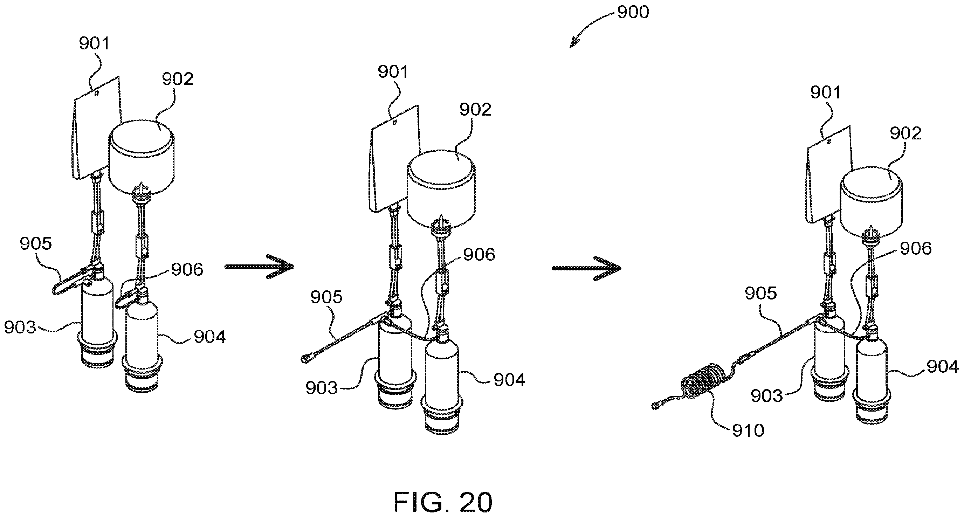

Referring to **FIG. 20**, a fluid transfer assembly **900** in accordance with another alternative example of the present disclosure is shown. The fluid transfer assembly **900** has a first container (e.g., a saline bag) **901** and a second container (e.g., a bottle of contrast agent) **902** liquidly coupled to respective first and second syringes **903, 904.** Once again, the details of how the first and second syringes **903, 904** are filled is substantially similar to that disclosed above with respect to **FIGS. 18A-18E****,** so further description of the fill process will be omitted herein. During the fill process, a connector tube **905** forms a passageway between the first syringe **903** and a connector tube leading to the first container **901,** while a separate tube **906** provides the passageway between the second syringe **904** and a connector tube leading to the second container **902.** After the filling of the first and second syringes **903, 904,** the connector tube **906** may be coupled to the connector tube **905.** With this fluid connection, the respective liquids within the first and second syringes **903, 904** may be combined together or delivered sequentially during the injection process. A separate low-pressure connector tube **910** may then be coupled to the connector tube **905,** primed, and provided to the patient via a wet connection to the connector tube **910.**

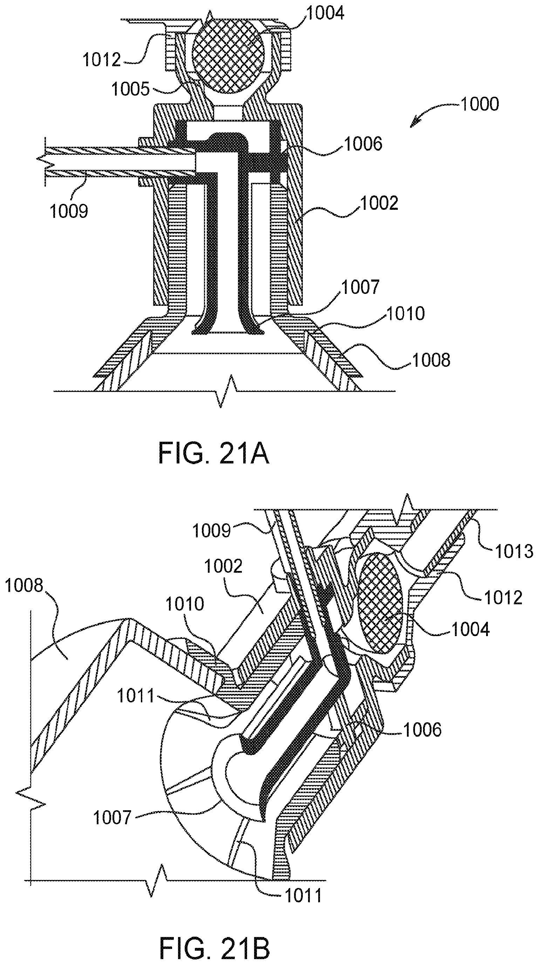

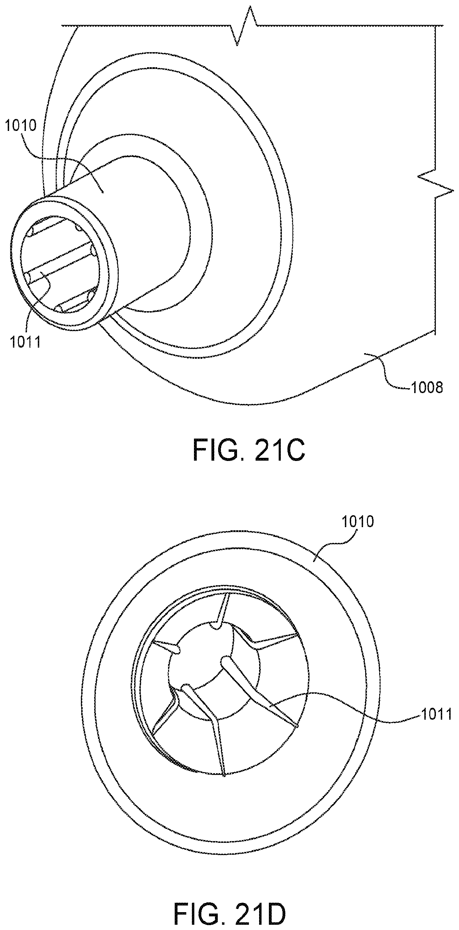

Referring now to **FIGS. 21A-21F****,** a fluid transfer assembly **1000** in accordance with another example of the present disclosure is illustrated. In particular, the fluid transfer assembly **1000** shows greater detail regarding a flow controller **1006** held within a valve housing **1002.** Similar to previous examples discussed above, the fluid transfer assembly **1000** has a valve housing **1002** having a connector cap **1012** coupled thereto, with an optional floating ball **1004** retained therein. Liquid is able to enter the valve housing **1002** via a connector tube **1013** connected to a container (not shown) positioned thereabove. The floating ball **1004** enables liquid to pass thereby by resting on a surface **1005** having liquid passages therein. The flow controller **1006** is further coupled to a connector tube **1009,** which allows air and/or liquid to pass therethrough. During an injection procedure where the liquid is delivered from the syringe **1008** under pressure, the floating ball **1004** seats against the distal end of the valve housing **1002** to prevent the flow of liquid into the liquid container.

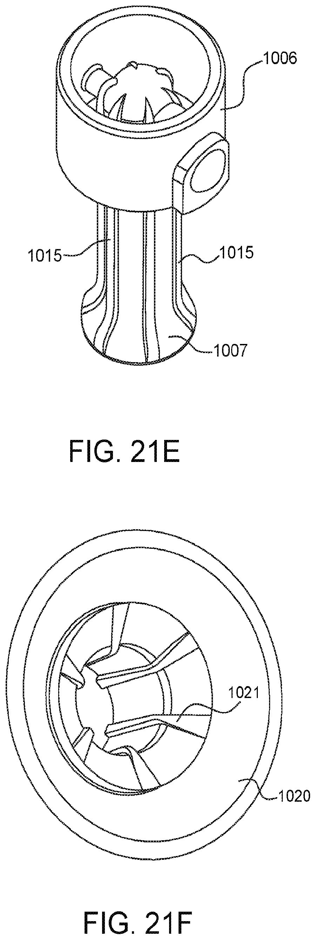

As discussed above, the flow controller **1006** is sized and shaped so as to direct the liquid flowing thereon to adhere to the inner surface of the syringe **1008** in accordance with the Coand 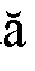 effect. To achieve flow in accordance with the Coand 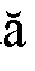 effect, it may be advantageous to provide ribs or contours on one or both of the external surfaces of the flow controller **1006** and a ribbed connector **1010.** For example. **FIGS. 21C-21D** illustrate the ribbed connector **1010,** with a plurality of rounded ribs **1011** formed on an inner surface thereon. As liquid passes through ribbed connector **1010,** the ribs **1011** may direct the liquid flow so as to encourage adhesion of the liquid to the inner surfaces of the syringe **1008.** Likewise, the flow controller **1006** may be sized and shaped to further induce such liquid flow. As shown in **FIG. 21E****,** the flow controller **1006** may have a plurality of ribs **1015** thereon, which similarly encourage uniform liquid flow. The ribs **1015** may have equal or unequal angular spacing around a longitudinal axis of the flow controller **1006.** The ribs **1015,** coupled with a flared end **1007,** may further encourage adhesion of the liquid to the inner surfaces of syringe **1008.** Also, while the ribbed connector **1010** is shown having rounded ribs **1011,** it may also be advantageous to have ribs of a different shape to control flow. For example, as **FIG. 21F** shows, a ribbed connector **1020** may have a plurality of squared ribs **1021** having flared ends to encourage liquid flow along the inner surfaces of the syringe **1008.** With some examples, a ratio of a cross-sectional area of the flared end **1007** to a cross-sectional area of an internal conduit of the flow controller **1006** may be between 5:1 to 20:1. With further examples, a radius of the flared end **1007** is desirably less than or equal to a radius of the sidewall of the syringe **1008** at a transition between the frusto-conical distal end and the syringe neck.

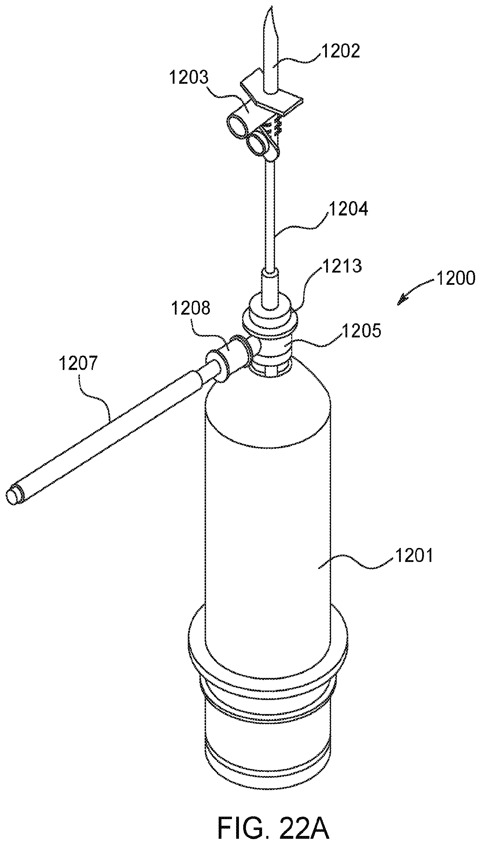

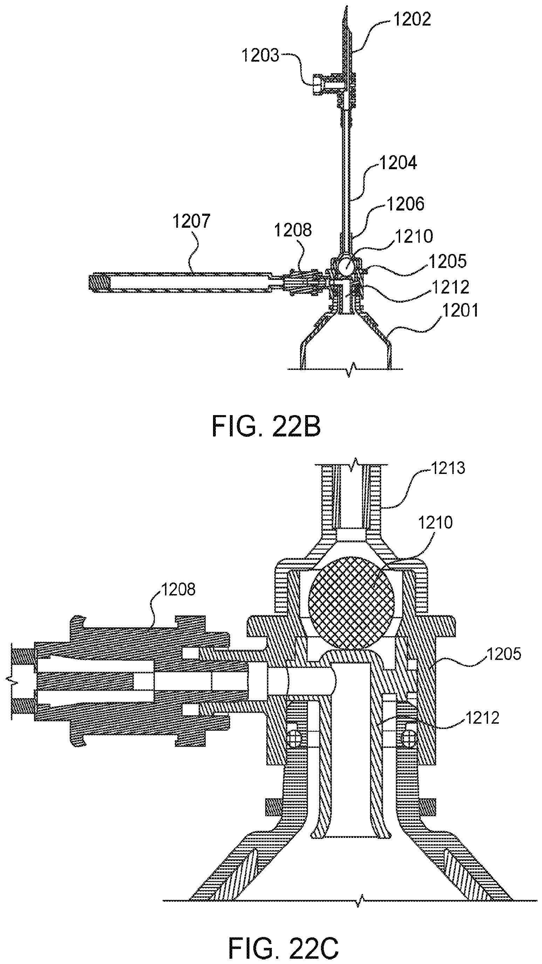

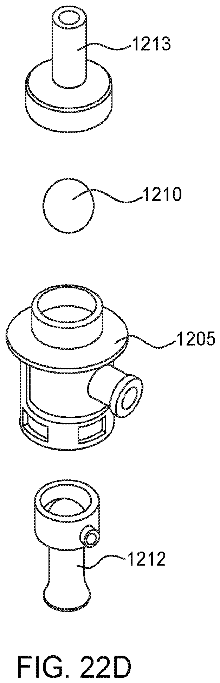

Next, referring to **FIGS. 22A-22D****,** a fluid transfer assembly **1200** in accordance with another example of the present disclosure is shown. The fluid transfer assembly **1200** has a syringe **1201** and a spike adapter **1202** configured for connection to a liquid container (not shown) above syringe **1201.** The spike adapter **1202** has a vent **1203** to allow air into the liquid container during the filling process. Connecting the syringe **1201** and the spike adapter **1202** is a fill tube **1204.** A valve housing **1205** is coupled to the open end of the syringe **1201,** with the valve housing **1205** having a connecting cap **1206** thereon and an optional floating ball **1210** retained therein. The valve housing **1205** is further configured to liquidly connect to a prime straw **1207** via a fitting **1208.** When the prime straw **1207** is removed, a separate tube (not shown) may be connected to the valve housing **1205** for eventual fluid connection to the patient after priming.

The valve housing **1205** is shown coupled to the syringe **1201,** with the fill tube **1204** liquidly coupled to the valve housing **1205** via the connecting cap **1206.** Also within the valve housing **1205** is a bell-shaped flow controller **1212.** Again, the bell-shaped contour of the surface of the flow controller **1212** directs the liquid along the interior walls of syringe **1201** in accordance with the Coand 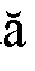 effect. A central opening in the flow controller **1212** provides a wide channel for air to flow from the syringe **1201** to the prime tube **1207,** thereby reducing the velocity of the air moving past the air/liquid interface. In this manner, slow moving air is unlikely to pull liquid in a distal direction, for example under a Bernoulli effect.

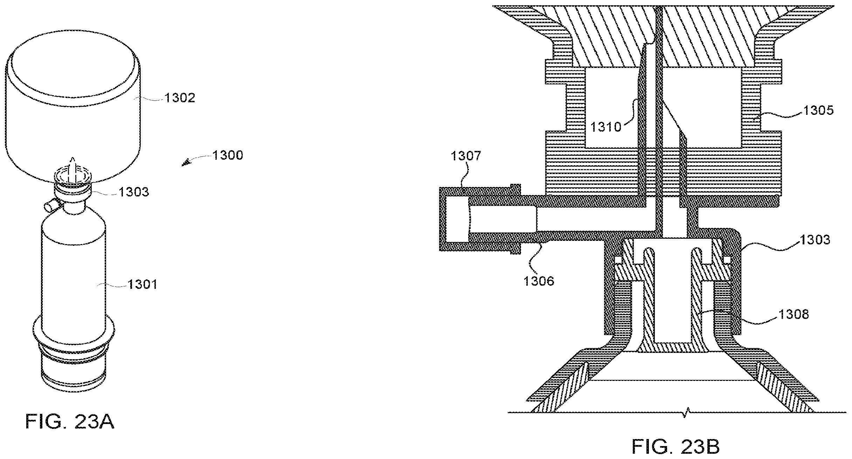

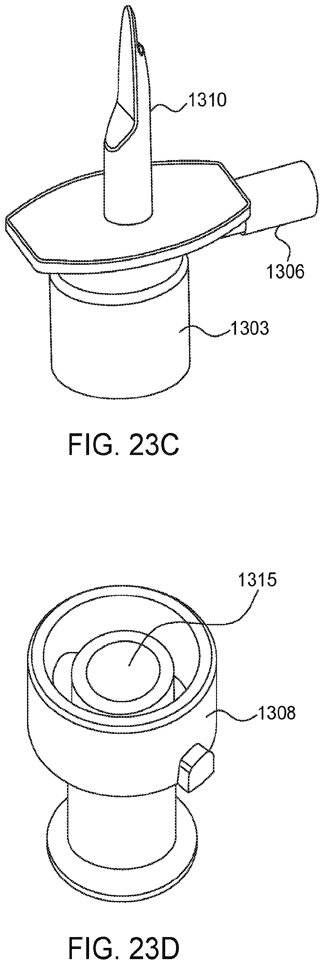

Referring now to **FIGS. 23A-23D****,** a fluid transfer assembly **1300** in accordance with another example of the present disclosure is shown. The fluid transfer assembly **1300** has a syringe **1301,** a container **1302,** and a spike adapter **1303** configured to directly liquidly connect the syringe **1301** and the container **1302.** Specifically, referring to **FIG. 23B****,** the spike adapter **1303** has a spike **1310** capable of piercing a septum or other sealed component of the container **1302.** The spike **1310** has two passages formed therethrough, one in fluid connection with the syringe **1301,** the other capable of venting air through vent conduit **1306** formed on the spike adapter **1303** into the container **1302,** with the vent conduit **1306** being coupled to a vent cap **1307.** As liquid passes through the spike **1310** from the container **1302,** for example during a vacuum fill procedure, it contacts a flow controller **1308.** As detailed in the examples above, the flow controller **1308** is formed so as to direct the liquid flow along the inner surfaces of syringe **1301** during the filling process. As shown in **FIG. 23B** and **FIG. 23D****,** specifically, the flow controller **1308** may have an inner opening **1315** formed therein, wherein any liquid entering the flow controller **1308** is urged to flow down the peripheral surfaces of the flow controller **1308** to better direct liquid flow along the inner surfaces of syringe **1301.**

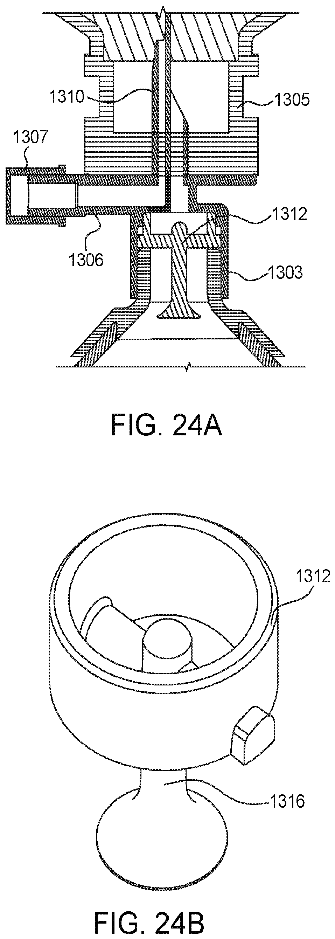

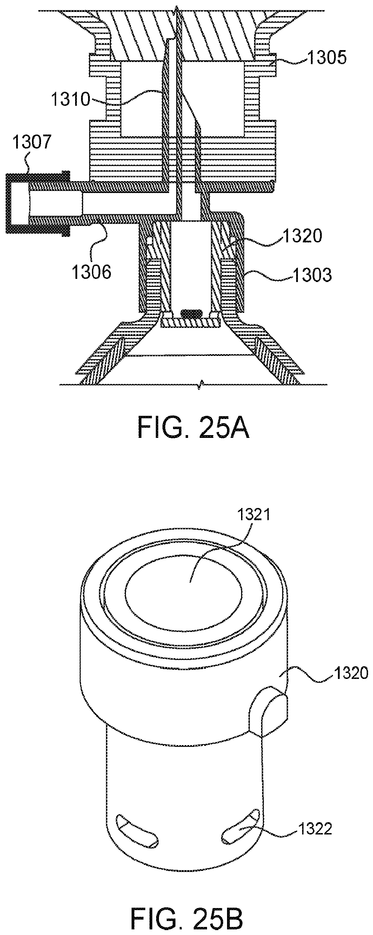

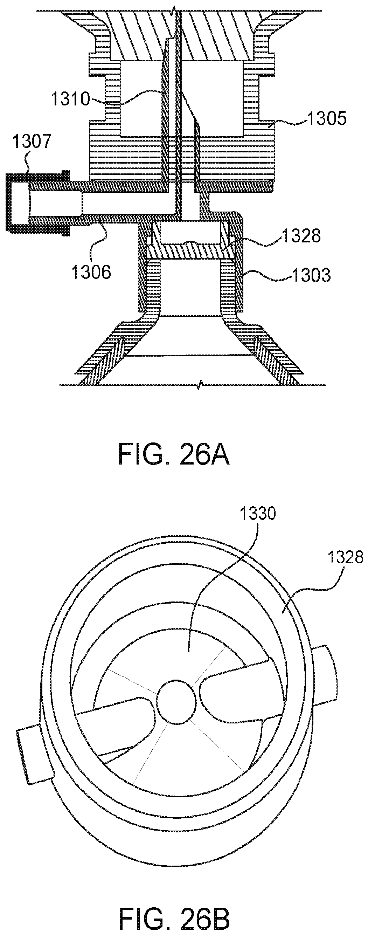

While the flow controller **1308** is shown to have an inner opening **1315** formed therein, the flow controller shape and contours in accordance with the disclosure are not limited to such. For example, **FIGS. 24A-24B****,** **FIGS. 25A-25B****,** and **FIGS. 26A-26B** illustrate alternative flow controllers in accordance with alternative examples of the present disclosure. In particular, **FIGS. 24A-24B** show a flow controller **1312** having a solid, bell-shaped component **1316** extending therebelow, with the bell-shaped component **1316** configured to direct liquid flow along the inner surfaces of the syringe **1301.** **FIGS. 25A-****25B** show a flow controller **1320** having an opening **1321** therein, with a bottom portion of the flow controller **1320** having a plurality of openings **1322** formed therein so as to allow the liquid to pass therethrough at or near the inner surfaces of the syringe **1301.** Further, **FIGS. 26A-26B** show a flow controller **1328** having a dome-shaped insert **1330** retainer therein to encourage the liquid passing therethrough to run along the inner surfaces of the syringe **1301.** The insert **1330** may be made from an elastomeric material such that an opening formed by deflecting at least a portion of the insert **1330** as a result of liquid flow can be adjusted.

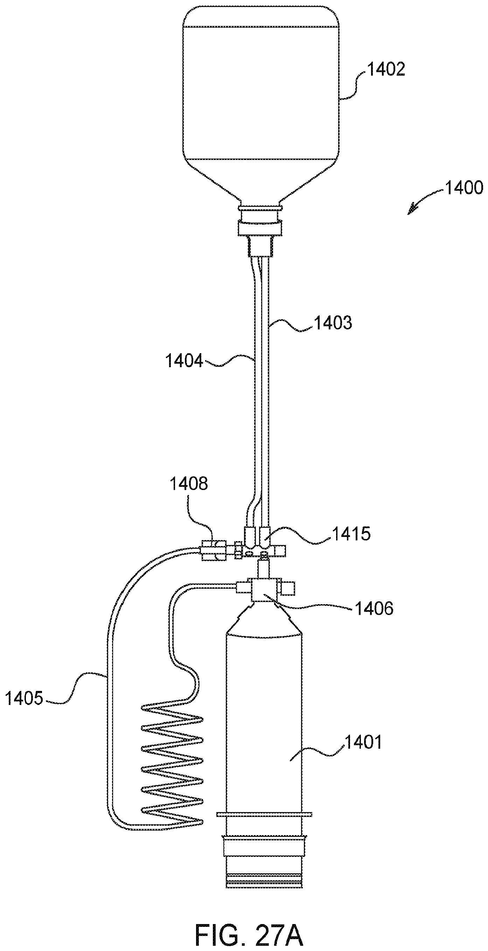

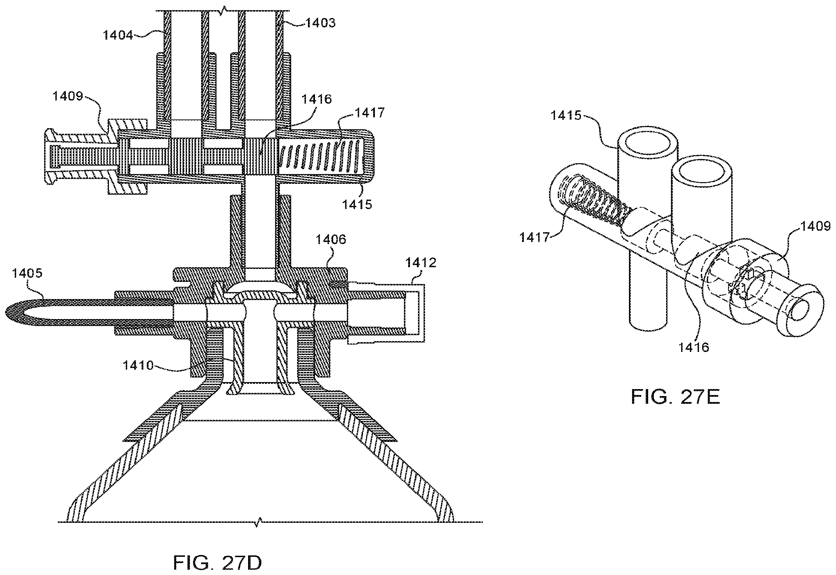

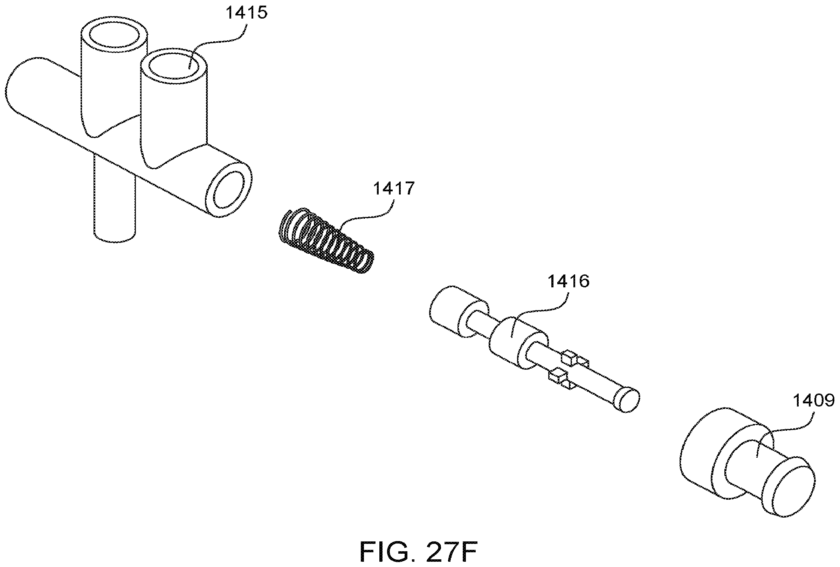

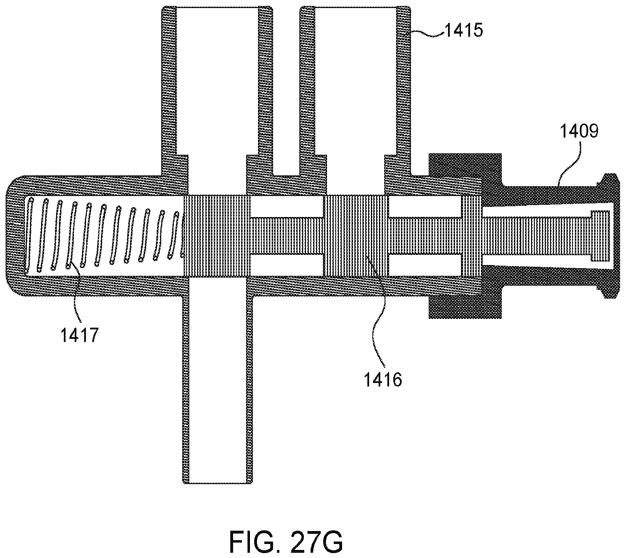

Referring now to **FIGS. 27A-27G****,** a fluid transfer assembly **1400** in accordance with another example of the present disclosure is shown. Many aspects of the fluid transfer assembly **1400** are similar to those previously discussed with respect to earlier fluid transfer assemblies, but the fluid transfer assembly **1400** utilizes a spool valve **1415** to enable or disable flow to/from a syringe **1401,** as will be discussed herein.

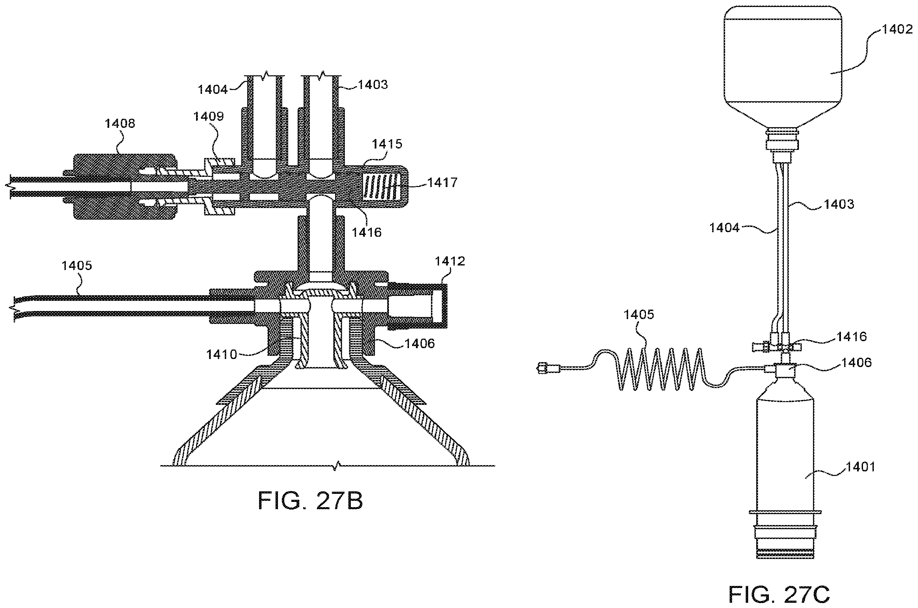

Specifically referring to **FIGS. 27A-27G****,** the fluid transfer assembly **1400** has a container **1402** and the syringe **1401,** with the container **1402** and the syringe **1401** liquidly coupled via dual respective connector tubes **1403, 1404.** A valve housing **1406** is coupled to the syringe **1401,** with the valve housing **1406** having a flow controller **1410** therein, a vent cap **1412** configured to vent air to/from the syringe **1401,** and a fitting for fluid connection to a low-pressure connector tube **1405.** The valve housing **1406** is further liquidly connected to a spool valve **1415,** such that the spool valve **1415** enables or disables flow of liquid through the valve housing **1406** and into the syringe **1401.** More specifically, the spool valve **1415** is liquidly connected to both connector tubes **1403, 1404.** During a syringe filling operation, one end of the connector tube **1405** is coupled to the valve housing **1406,** while a second end of the connector tube **1405,** having a fitting **1408,** is coupled to the spool valve **1415** at a fitting **1409.** Within the spool valve **1415** is a valve member **1416** configured to be longitudinally biased to a "closed" position via a spring **1417** or other biasing member. However, when the fitting **1408** of the connector tube **1405** is coupled to the fitting **1409,** a portion of the fitting **1408** urges valve member **1416** from a "closed" position (shown in **FIG. 27D**) to the "open" position (shown in **FIG. 27B****),** thereby allowing liquid to flow from the connector tube **1403** into the syringe **1401,** and further allowing air to from the syringe **1401** to pass through the connector tube **1405** and into the connector tube **1404** for passage to the container **1402.** In this manner, the fluid transfer assembly **1400** is a closed system where no air from outside the fluid transfer assembly **1400** is introduced. Rather, the air exchange between the container **1402** and the syringe **1401** during a filling operation of the syringe **1401** is handled by the connector tubes **1403, 1404,** and **1405,** and only sterile air within the fluid transfer assembly **1400** is exchanged. The closed system of the fluid transfer assembly **1400** may be suitable for transferring liquids where prevention of contamination of the liquid by outside air is desired, such as with chemotherapy drugs.

Referring to **FIGS. 27C-27D****,** when the fitting **1408** is removed from the spool valve **1415,** the valve member **1416** is no longer urged against the spring **1417.** Accordingly, the spring **1417** biases the valve member **1416** to the "closed" position **(****FIG. 27D****),** thereby preventing the flow of liquid from the connector tube **1403** into the syringe **1401,** and also blocking the passage of air or liquid out of the connector tube **1404.**

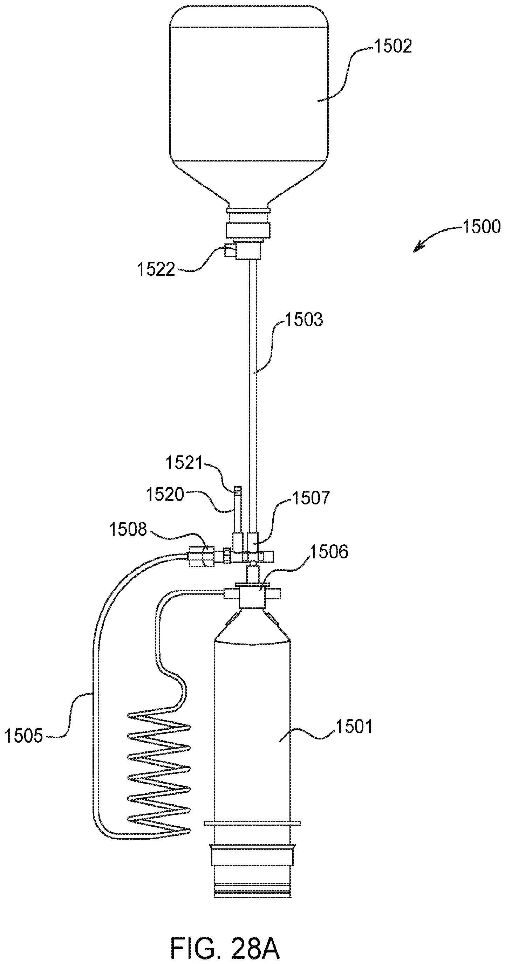

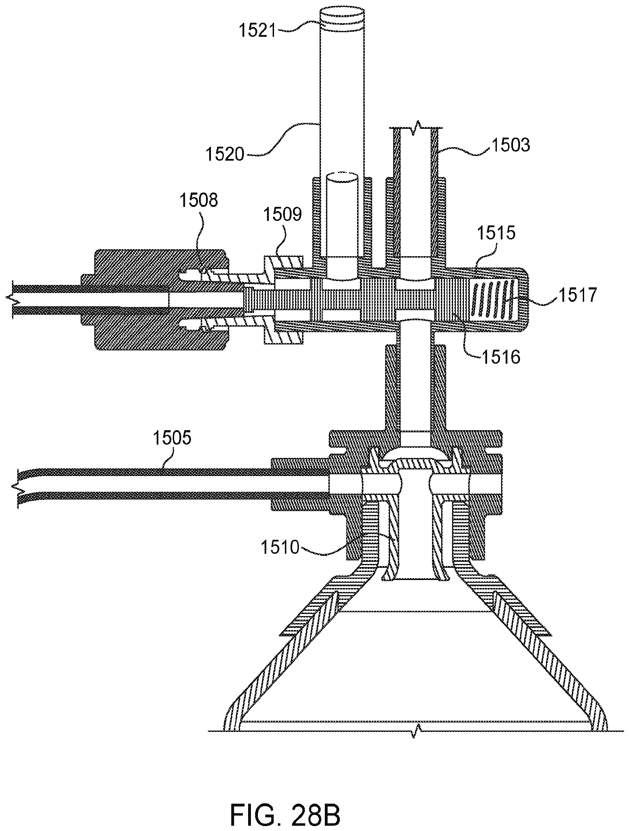

Referring to **FIGS. 28A-28B****,** a fluid transfer assembly **1500** in accordance with another aspect of the present disclosure is shown. Many aspects of the fluid transfer assembly **1500** are similar to those previously discussed above with respect to the fluid transfer assembly **1400,** but the fluid transfer assembly **1500** does not require the use of a secondary tube (such as the tube **1404** in **FIGS. 27A-27G**) extending between a syringe **1501** and a container **1502,** as described herein.

As shown in **FIGS. 28A-28B****,** the fluid transfer assembly **1500** has the container **1502** and the syringe **1501,** with the container **1502** and the syringe **1501** liquidly coupled via a single connector tube **1503.** The connector tube **1503** is liquidly coupled to the container **1502** via a vented coupler **1522,** which has an air filter therein so as to allow air within the container **1502** to vent during transfer of liquid between the container **1502** and the syringe **1501.** A valve housing **1506** is coupled to the syringe **1501,** with the valve housing **1506** having a flow controller **1510** therein, and a fitting for fluid connection to a low-pressure connector tube **1505.** The valve housing **1506** is further liquidly connected to a spool valve **1515,** such that the spool valve **1515** enables or disables flow of liquid through the valve housing **1506** and into the syringe **1501.** More specifically, the spool valve **1515** is liquidly connected to the connector tube **1503.** During a syringe filling operation, one end of the connector tube **1505** is coupled to the valve housing **1506,** while a second end of the connector tube **1505,** having a fitting **1508,** is coupled to the spool valve **1515** at a fitting **1509.** Within the spool valve **1515** is a valve member **1516** configured to be longitudinally biased to a "closed" position via a spring **1517.** However, when the fitting **1508** of the connector tube **1505** is coupled to the fitting **1509,** a portion of the fitting **1508** urges the valve member **1516** to the "open" position, thereby allowing liquid to flow from the connector tube **1503** into the syringe **1501.** The valve member **1516** may be solid or hollow. In a hollow configuration, the valve member **1516** may allow air to pass through an inner portion of the valve **1515,** while liquid flowing through the connector tube **1503** into the syringe **1501** is allowed to pass over the outer surfaces thereof.

In addition to the valve member **1516** allowing liquid to pass between the container **1502** and the syringe **1501** when in the "open" configuration, the valve member **1516** also enables air to flow from the syringe **1501,** through the connector tube **1505,** and out of a vented air filter **1521** located on a prime tube **1520** coupled to the spool valve **1515.** Specifically, during transfer of liquid from the container **1502** into the syringe **1501,** air is purged out of the syringe **1501** through connector tube **1505.** The air filter **1521,** which can be, for example, a 0.2 micron air filter, allows the purged air to vent to atmosphere. When the fitting **1508** is removed from the spool valve **1515,** the valve member **1516** is no longer urged against the spring **1517.** Accordingly, the spring **1517** biases the valve member **1516** to the "closed" position, thereby preventing the flow of liquid from the connector tube **1503** into the syringe **1501.** In the "closed" position, the valve member **1516** may also block the passage of air or liquid out of the air filter **1521,** but it may alternatively allow the air passage to remain open when disconnected from the connector tube **1505.**

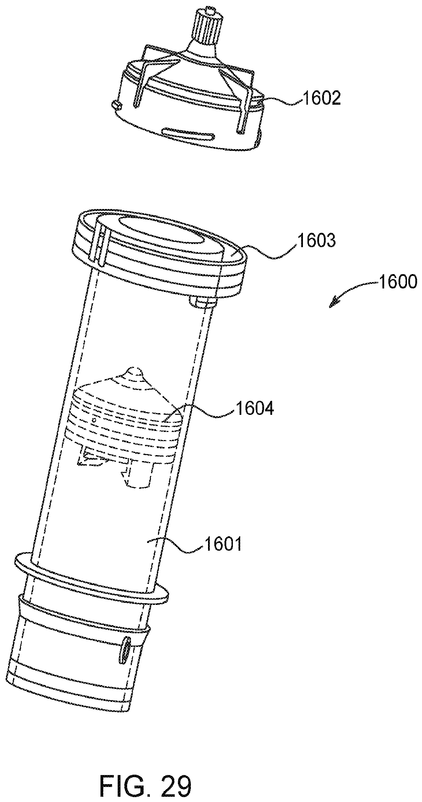

With reference to **FIG. 29****,** a syringe **1600** in accordance with another aspect of the disclosure is shown. The syringe **1600** is configured to receive a flexible rolling diaphragm, such as the rolling diaphragm syringe **12a** shown in **FIG. 2A****,** within an interior space of the syringe **1600.** The syringe **1600** has a base adapter **1601** having a plunger **1604** disposed therein, as well as a connector **1603** on a distal end thereof. A syringe tip **1602** is configured for removable connection with the connector **1603** via, for example, a threaded connection. The syringe tip **1602** is desirably a disposable, single-use item. However, the base adapter **1601** is a multi-use item, capable of use between many procedures and/or patients. Accordingly, instead of the entire syringe assembly being single-use (as is currently common), the syringe **1600** enables only the syringe tip **1602** to be replaced between uses. Furthermore, different types of syringe tips could be utilized with one type of base adapter.

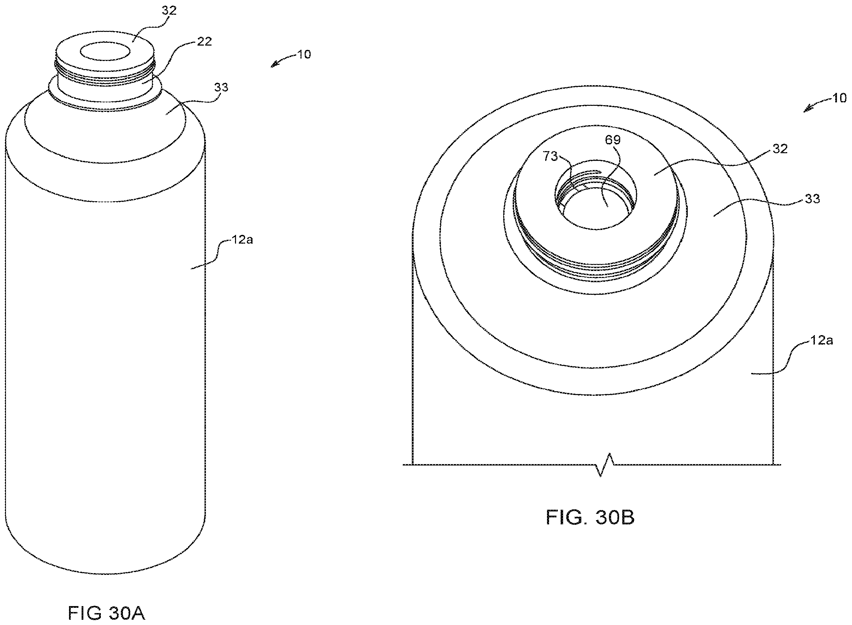

With reference to **FIGS. 30A-30C****,** a fluid transfer assembly **10** is illustrated in accordance with another example of the present disclosure. The fluid transfer assembly **10** is configured to facilitate transfer of liquid from a second liquid container, such as a bottle or a bag (not shown), to a first liquid container, such as a syringe **12a** described herein with reference to **FIG. 2A****.** In other examples, the first liquid container may be the syringe **12b** described herein with reference to **FIG. 2B****.**

With continued reference to **FIGS. 30A-30C****,** the fluid transfer assembly **10** includes a fill adapter **32** configured for facilitating a transfer of liquid between the two liquid containers, such as from the second liquid container to the syringe **12a.** The fill adapter **32** may be removably connectable to the syringe **12a,** such as the syringe neck **22** of the syringe **12a.** In some examples, the fill adapter **32** may be non-removably connected to the syringe **12a,** such as by being monolithically formed therewith, or by being non-removably attached thereto, such as by adhesive, welding, interference fit, or other mechanical connection means. The fill adapter **32** may have a unitary, single piece structure, or it may be formed from two or more components removably or non-removably coupled together. In various aspects, fill adapter **32** may flow between 2 mL/s to 20 mL/s of liquid while filling syringe **12a.**

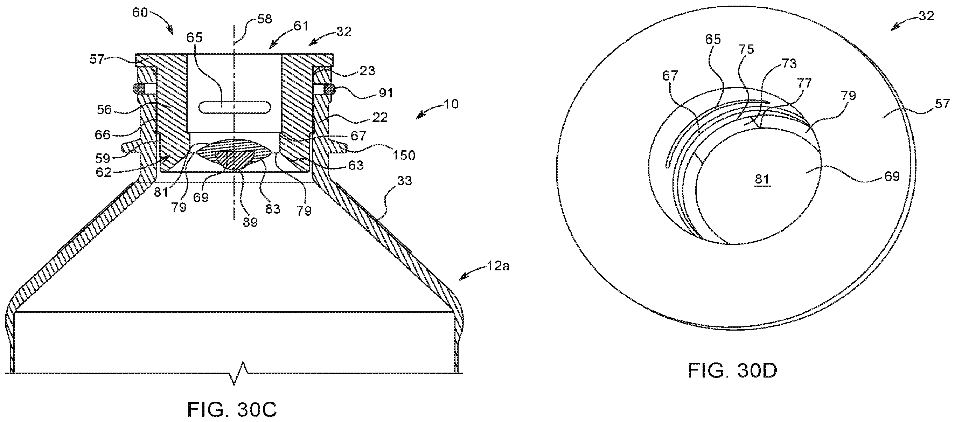

With reference to **FIG. 30C****,** the fill adapter **32** has a substantially cylindrical body **56** that is configured to be received within the open distal end **23** of the syringe neck **22.** A flange **57** extends radially outward from the body **56** at a distal end **60.** In some examples, an outer diameter of the flange **57** may substantially correspond to an outer diameter of the syringe neck **22** such that the fill adapter **32** is flush with the syringe neck **22.** In other examples, the outer diameter of the flange **57** may be larger or smaller than the outer diameter of the syringe neck **22.** A longitudinal length of the body **56** along the longitudinal axis **58** may correspond to a longitudinal length of the syringe neck **22** such that a proximal end **62** of the fill adapter **32** terminates at a proximal end of the syringe neck **22** and before the syringe sidewall **20** transitions from the syringe neck **22** to a conical portion **33.** In some examples, the longitudinal length of the body **56** may be longer or shorter than a transition point where the syringe sidewall **20** transitions from the syringe neck **22** to the conical portion **33.** In various examples, the longitudinal length of body **56** may be between 6 mm to 100 mm.

The fill adapter **32** may be dimensioned such that it fits snuggly within the open distal end **23** of the syringe neck **22.** For example, an outer sidewall **66** of the fill adapter **32** may be engaged with an inner sidewall of the syringe neck **22** due to an interference fit between an outer sidewall **66** of the fill adapter **32** and an inner sidewall **26** of the syringe neck **22.** The fill adapter **32** may be removably or non-removably engaged with the syringe neck **22.** In some examples, the fill adapter **32** and the syringe neck **22** may be removably or non-removably connected to one another by way of clips, fasteners, adhesive, welding, or other mechanical connection means. In some examples, an outer surface of the body **56** may have one or more recesses **59** to facilitate insertion of the fill adapter **32** into the open distal end **23** of the syringe neck **22.** In various examples, an outer diameter of the body **56** of the fill adapter may be between 2.6 mm and 26 mm.

With continued reference to **FIG. 30C****,** the fill adapter **32** has a central bore **61** extending through the body **56** along the longitudinal axis **58.** The central bore **61** is configured for allowing liquid communication between the second liquid container and the interior of the syringe **12a.** In some examples, the central bore **61** may have a uniform diameter throughout the length thereof. In other examples, at least a portion of the central bore **61** may narrow or widen in a direction from the distal end **60** to the proximal end **62** of the body **56.** For example, an angled portion **63** of the central bore **61** may widen in a direction from the distal end **60** to the proximal end **62** of the body **56,** such as shown in **FIG. 30C****.** In some examples, an angle of the angled portion **63** may correspond to an angle of the conical portion **33** of the syringe **12a.** In other examples, the angle of the angled portion **63** may have a larger or smaller angle than the angle of the conical portion **33** of the syringe **12a.** In various examples, an angle of the angled portion **63** may be between 10 degrees and 80 degrees relative to the longitudinal axis of the body **56.**

One or more slots **65** may extend through the sidewall of the body **56.** In some examples, a plurality of slots **65** may extend through the sidewall of the body **56** at equal or unequal angular intervals therebetween. The slots **65** may be provided at a same axial position relative to one another, or one or more of the slots **65** may be offset axially relative to the remaining slots **65.** In some examples, a ledge **67** may protrude radially inward from an inner surface of the central bore **61.** The ledge **67** may be positioned proximally or distally of the one or more slots **65.** The ledge **67** may be continuous or discontinuous in a circumferential direction of the central bore **61.** The slots **65** and/or the ledge **67** interrupt the flow of fluid along an inner sidewall of the central bore **61.** In this manner, the slots **65** and/or the ledge **67** assist in guiding the fluid flowing through the fill adapter **32** toward a central portion of the bore **61** and away from the inner sidewall of the bore **67** such that the fluid can flow over a flow controller **69,** as described herein.

With continued reference to **FIG. 30C****,** the fill adapter **32** may have the flow controller **69** disposed within the central bore **61.** The flow controller **69** may be positioned at the proximal end **62** of body **56.** In some examples, the flow controller **69** is connected to an inner surface of the central bore **61** by one or more spokes **73** (shown in **FIG. 30D**). As shown in **FIG. 30D****,** each of the spokes **73** has a first end **75** connected to the body **56** of the fill adapter **32** and a second end **77** connected to the flow controller **69.** A gap **79** between the body **56** of the fill adapter **32** and the flow controller **69** is configured to allow liquid to flow therethrough and into the interior of the syringe **12a.**

The flow controller **69** may be positioned such that an outer edge of the flow controller **69** is aligned with a distal end of the angled portion **63.** In this manner, liquid that is deflected by the flow controller **69** will be deflected toward the angled portion **63.** As described herein, due to the characteristics of the Coand 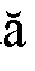 effect, liquid will be attracted to the angled surface of the angled portion **63** of the fill adapter **32** and will continue flowing down the inner sidewall of the syringe **12a.**

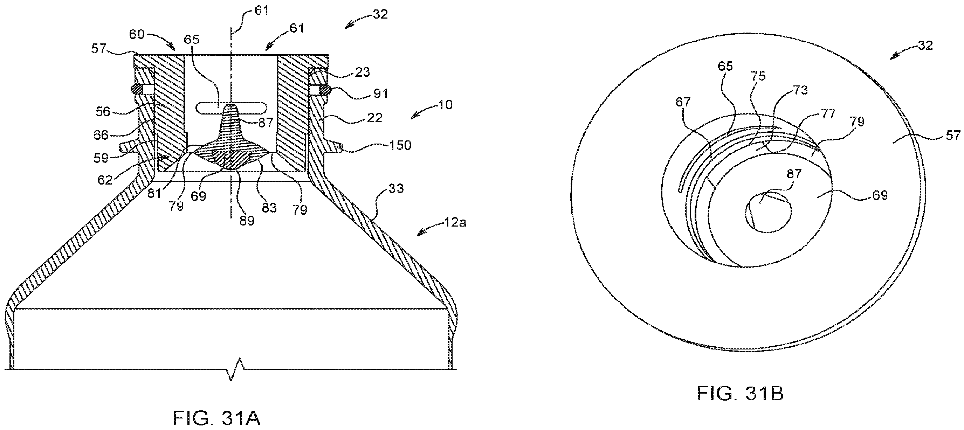

With reference to **FIG. 30D****,** and with continued reference to **FIG. 30C****,** the flow controller **69** may have a curved distal surface **81** that is configured to divert liquid flowing onto the flow controller **69** in a radially outward direction toward the gap **79.** The distal surface **81** may have a convex shape having uniform or non-uniform curvature. In some examples, the flow controller **69** may have a curved proximal surface **83.** The proximal surface **83** may have a convex shape with a protrusion **89** in a central portion thereof. The protrusion **89** may protrude in a proximal direction from the proximal surface **83.** In some examples, such as shown in **FIGS. 31A-31B****,** a central portion of the distal surface **81** may have a flow diverter **87.** The flow diverter **87** may extend distally relative to the distal surface **81.** The flow diverter **87** may be configured to divert the liquid flowing through the central bore **61** of the fill adapter **32** toward outer edges of the flow controller **69** such that the liquid flows through the gap **79.**

In some examples, the syringe neck **22** may have a sealing member, such as an O-ring **91.** The O-ring **91** is configured to sealingly engage a cap (not shown) removably connectable to the syringe neck **22** of the syringe **12a.** An outer portion of the syringe neck **22** may have a flange **150** configured for interacting with the cap for removably retaining the cap with the syringe neck **22.** In some examples, the fill adapter **32** may be removably or non-removably connected to the cap such that the fill adapter **32** is removable from the syringe **12a** with the removal of the cap. In other examples, the fill adapter **32** is provided separate from the cap such that the fill adapter **32** remains connected to the syringe **12a** when the cap is removed from the syringe **12a.**

During the filling process, a liquid from the second liquid container (not shown) above the syringe **12a** is gravity-fed or vacuum-fed to the fill adapter **32.** As the liquid flows through the central bore **61** of the fill adapter **32,** the liquid contacts the flow controller **69.** Due to the convex shape of the distal surface **81** of the flow controller **69,** the liquid will be directed radially outward to flow through the gap **79.** Due to the bell-shaped angled portion **63,** the liquid is naturally attracted to flow along the conical portion **33** of the syringe **12a** before flowing down the sidewall **20**. This flow along the inside surface of the syringe **12a** helps to reduce turbulence as the liquid fills the syringe **12a,** which aides in reducing air bubbles from forming as the syringe **12a** is filled. Simultaneously, air is displaced from the syringe interior and escapes into the second fluid container through the gap **79.** Air/liquid exchange within the syringe **12a** occurs substantially simultaneously through the central bore **61,** without any flow hesitation or gurgling due to uneven flow characteristics.

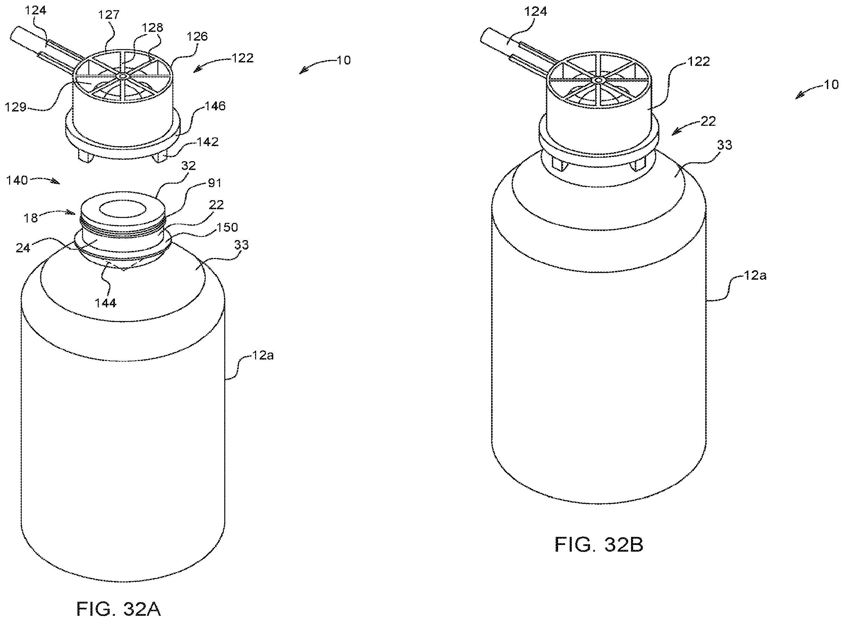

With reference to **FIGS. 32A-32D****,** a cap **122** may be provided on the syringe neck **22** of the syringe **12a.** The cap **122** is configured to enclose the distal end **18** of the syringe **12a.** The cap **122** is removably connected to the syringe neck **22** of the syringe **12a,** for example via a threaded connection, a snap-fit connection, a friction-fit connection, or any other suitable removable connection mechanism. In some examples, the fill adapter **32** may be removably or non-removably connected to the cap **122** such that the fill adapter **32** is removable from the syringe **12a** with the removal of the cap **122.** In other examples, the fill adapter **32** is provided separate from the cap **122** such that the fill adapter **32** remains connected to the syringe **12a** when the cap **122** is removed from the syringe **12a.**

The cap **122** includes a port **124** configured for connection to tubing that is in liquid communication with a second liquid container (not shown) for filling the syringe **12a** with liquid from the second liquid source. In other examples, the port **124** may be configured for connection to tubing connected to a catheter, needle, or other liquid delivery connection (not shown) inserted into a patient at a vascular access site to deliver liquid from the syringe **12a** to the patient.

In some examples, the port **124** may be provided on a radial side of the cap **122.** A high crack pressure valve (not shown) may be provided on the cap **122** to permit flow of liquid through the cap **122** into the syringe **12a** when a predetermined crack pressure is reached or exceeded, while preventing flow through the cap **122** when the head height of pressure is less than the crack pressure. In one example, the high crack pressure valve may be a slit valve or other conventional crack pressure valve. The cap **122** may have at least one gasket around an inner surface of the cap **122** for engaging an outer surface **24** of the syringe neck **22.** In some examples, the gasket, such as the O-ring **91,** may be provided on the outer surface **24** of the syringe neck **22.** The O-ring **91** is configured to create a liquid-tight seal between the outer surface **24** of the syringe neck **22** and the inner surface of the cap **122.** With reference to **FIGS. 32A-32B****,** a distal surface **126** of the cap **122** may be configured as a substantially flat surface.

With continued reference to **FIGS. 32A-32B****,** the cap **122** is removably connectable to the syringe **12a** by way of a connection mechanism **140.** The connection mechanism **140** is configured to securely retain the cap **122** connected to the syringe neck **22** of the syringe **12a,** such as shown in **FIG. 32B****,** and allow the cap **122** to be disconnected from the syringe neck **22** of the syringe **12a** with rotation of the cap **122** about its longitudinal axis relative to the syringe **12a.** The connection mechanism **140** has one or more tabs **142** on the cap **122** that interact with one or more cams **144** on the syringe **12a.** When the cap **122** is connected to the syringe **12a,** rotation of the cap **122** relative to the syringe **12a** causes the one or more tabs **142** to interact with the one or more cams **144** to deflect the tabs **142** radially outward such that the cap **122** can be removed from the syringe **12a.**

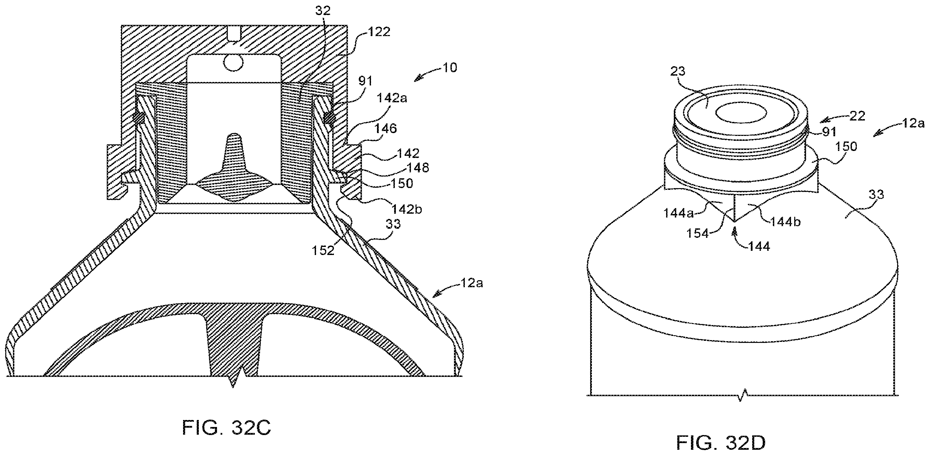

With reference to **FIG. 32C****,** the tabs **142** protrude in a proximal direction from a skirt **146** that extends around an outer circumference of the cap **122.** The tabs **142** may be spaced apart from one another at equal or unequal angular intervals around the skirt **146.** Each tab **142** has a first end **142a** connected to the skirt **146** and a second end **142b** protruding proximally from the skirt **146.** The second end **142b** of each tab **142** is deflectable relative to the first end **142a** when the tab **142** engages the cam **144.** The second end **142b** of each tab **142** has a recess **148** that is configured to engage a flange **150** extending radially outward from the outer surface **24** of the syringe neck **22.** The flange **150** may extend around a portion or an entire circumference of the syringe neck **22.** When engaged with the flange **150,** the recess **148** prevents the cap **122** from being removed from the syringe **12a.** Each tab **142** may have a beveled edge **152** that is configured to engage a distal surface of the flange **150** to deflect the tab **142** in a radially outward direction when the cap **122** is being connected to the syringe neck **22.** After the beveled edge **152** clears the flange **150,** each tab **142** can deflect back to engage the recess **148** with the flange **150,** thereby connecting the cap **122** with the syringe neck **22** of the syringe **12a.**

With reference to **FIG. 32D****,** the cams **144** are positioned proximally of the flange 150. In some examples, a plurality of cams **144** is spaced apart from one another around an outer circumference of the syringe neck **22** below the flange **150.** The cams **144** may be spaced apart from one another in equal or unequal angular intervals. For example, **FIG. 32D** shows a syringe **12a** with four cams **144** spaced apart from each other at 90 degrees. In this manner, rotation of the cap **122** of less than 90 degrees causes the tabs **142** to engage the cams **144** to disconnect the cap **122** from the syringe **12a.** Each cam **144** has a pair of ramp surfaces **144a, 144b** that come together at an edge **154.** In some examples, the edge **154** may terminate at an outer end of the flange **150.** The ramp surfaces **144a, 144b** define an engagement surface for the second end **142b** of the tabs **142** to contact when the cap **122** is to be disconnected from the syringe **12a.**

To remove the cap **122** from the syringe **12a,** the cap **122** can be rotated in a first direction (e.g., clockwise) or a second direction (e.g., counterclockwise) about its longitudinal axis relative to the syringe longitudinal axis. During such rotational movement of the cap **122,** the second end **142b** of each tab **142** engages one of the ramp surfaces **144a, 144b** of each cam **144.** Due to the angled configuration of the ramp surfaces **144a, 144b,** continued rotation of the cap **122** causes the second end **142b** of the tabs **142** to be deflected relative to the first end **142a** (and the flange **150**), thereby allowing the tabs **142** to move past the flange **150.** In this manner, the cap **122** can be disconnected from the syringe neck **22** with a simple rotation of the cap **122** relative to the syringe **12a.**

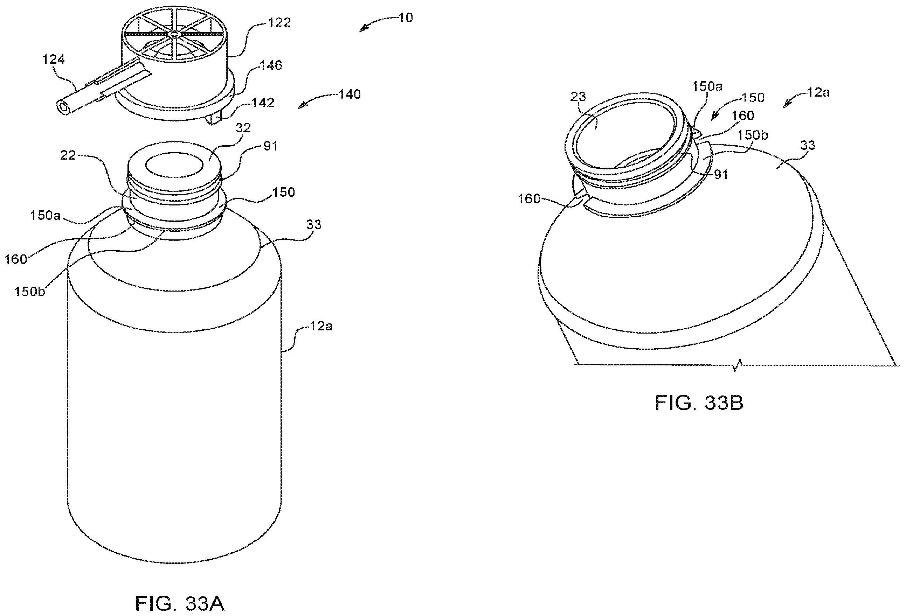

With reference to **FIGS. 33A-33C****,** a fluid transfer assembly **10** is shown in accordance with another example of the present disclosure. The components of the fluid transfer assembly **10** shown in **FIGS. 33A-33C** are substantially similar to the components of the fluid transfer assembly **10** described herein with reference to **FIGS. 32A-32D****.** As the previous discussion regarding the fluid transfer assembly **10** generally shown in **FIGS. 32A-****32D** is applicable to the examples shown in **FIGS. 33A-33C****,** only the relative differences between the two liquid transfer assemblies **10** are discussed hereinafter.

The cap **122** is removably connectable to the syringe **12a** by way of a connection mechanism **140.** In some examples, the fill adapter **32** may be removably or non-removably connected to the cap **122** such that the fill adapter **32** is removable from the syringe **12a** with the removal of the cap **122.** In other examples, the fill adapter **32** is provided separate from the cap **122** such that the fill adapter **32** remains connected to the syringe **12a** when the cap **122** is removed from the syringe **12a.** The connection mechanism **140** is configured to securely retain the cap **122** connected to the syringe neck **22** of the syringe **12a,** and allow the cap **122** to be disconnected from the syringe neck **22** of the syringe **12a** with rotation of the cap **122** about its longitudinal axis relative to the syringe **12a** to a removal position at which the cap **122** can be separated from the syringe **12a** with axial movement of the cap **122** relative to the syringe **12a.** The connection mechanism **140** has one or more tabs **142** on the cap **122** that interact with a flange **150** on the syringe **12a.**

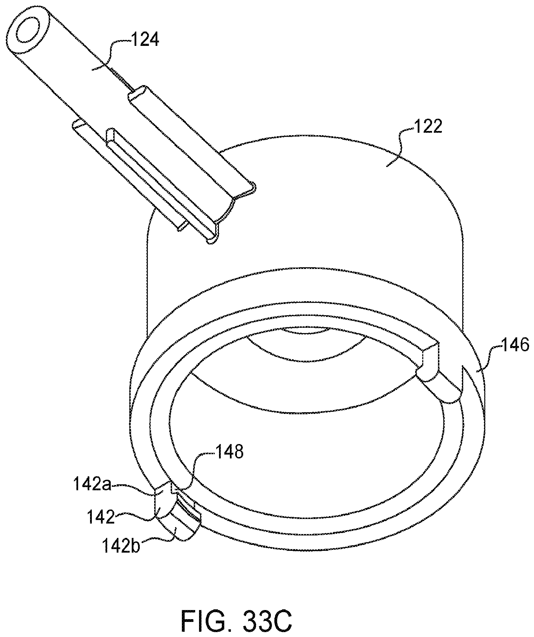

With reference to **FIG. 33C****,** the one or more tabs **142** on the cap **122** protrude in a proximal direction from a skirt **146** that extends around an outer circumference of the cap **122.** The tabs **142** may be spaced apart from one another at equal or unequal angular intervals around the skirt **146.** Each tab **142** has a first end **142a** connected to the skirt **146** and a second end **142b** protruding proximally from the skirt **146.** Desirably, the second end **142b** of each tab **142** is deflectable relative to the first end **142a.** The second end **142b** of each tab **142** has a recess **148** that is configured to engage the flange **150** extending radially outward from the outer surface **24** of the syringe neck **22** (shown in **FIG. 33B**).

With reference to **FIG. 33B****,** the flange **150** may extend around a portion of the outer circumference of the syringe neck **22.** In some examples, the flange **150** may be formed as a continuous member having a first end and a second end separated by a gap **160.** In other examples, the flange **150** may be formed from a plurality of segments separated from one another by two or more gaps **160.** For example, the flange **150** may have two flange segments **150a, 150b** separated by two gaps **160.** The flange segments **150a, 150b** may have equal or unequal length and may be separated by gaps **160** having equal or unequal width. Each gap **160** defines a removal position at which the tabs **142** can be aligned for removal of the cap **122** from the syringe neck **22,** as discussed herein. Desirably, the number of tabs **142** corresponds to the number of gaps **160.** When engaged with the flange **150,** the recess **148** prevents the cap **122** from being removed from the syringe **12a.** Each tab **142** may have a beveled edge **152** that is configured to engage a distal surface of the flange **150** to deflect the tab **142** in a radially outward direction when the cap **122** is being connected to the syringe neck **22.** After the beveled edge **152** clears the flange **150,** each tab **142** can deflect back to engage the recess **148** with the flange **150,** thereby connecting the cap **122** with the syringe neck **22** of the syringe **12a.**

To remove the cap **122** from the syringe **12a,** the cap **122** can be rotated in a first direction (e.g., clockwise) or a second direction (e.g., counterclockwise) about its longitudinal axis relative to the syringe longitudinal axis. During such rotational movement of the cap **122,** the tabs **142** can be rotated to the removal position by aligning the tabs **142** with the gaps **160** between the flange segments **150a, 150b.** When the cap **122** is rotated to the removal position, the gaps **160** create a clearance space for the tabs **142** to clear the flange segments **150a, 150b.** The cap **122** can be disconnected from the syringe neck **22** with axial movement of the cap **122** relative to the syringe **12a.**

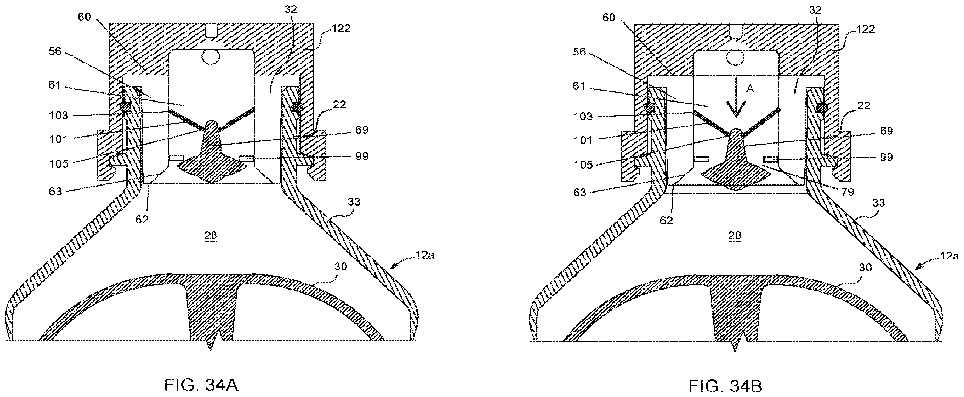

With reference to **FIG. 34A-34B****,** a fill adapter **32** is illustrated in accordance with another example of the present disclosure. The fill adapter **32** is configured to facilitate transfer of liquid from a second liquid container, such as a bottle or a bag (not shown), to a first liquid container, such as a syringe **12a.** The components of the fill adapter **32** shown in **FIG. 34** are substantially similar to the components of the fill adapter **32** described herein with reference to **FIGS. 30A-31B** except where noted. As the previous discussion regarding the fill adapter **32** generally shown in **FIGS. 30A-31B** is applicable to the example shown in **FIG. 34****,** only the relative differences between the two fill adapters **32** are discussed herein.

The fill adapter **32** is dimensioned such that it fits snuggly within the open distal end of the syringe neck **22,** such as due to engagement of an outer sidewall of the fill adapter **32** with an inner sidewall of the syringe neck **22.** A body **56** of the fill adapter **32** has a central bore **61** extending along a longitudinal axis. The central bore **61** is configured for allowing liquid communication between the second liquid container and the interior of the syringe **12a.** The bore **61** has an angled portion **63** that widens in a direction from a distal end **60** to a proximal end **62** of the body **56.** A sealing lip **99** may protrude radially inward from an inner surface of the central bore **61.** The sealing lip **99** may be positioned distally of the angled portion **63.** Desirably, the sealing lip **99** is continuous in a circumferential direction of the central bore **61.** In some examples, the sealing lip **99** may be made from the same or different material as the body **56.** For example, the sealing lip **99** may be made from a flexible elastomeric material, while the body **56** is made from a rigid plastic material.

The fill adapter **32** has a flow controller **69** disposed within the central bore **61.** The flow controller **69** may be positioned at the proximal end **62** and is connected to an inner surface of the central bore **61** by one or more resiliently elastic elements **101.** Each of the resiliently elastic elements **101** has a first end **103** connected to the body **56** of the fill adapter **32** and a second end **105** connected to the flow controller **69.** The flow controller **69** is movable axially in a direction of the longitudinal axis due to liquid flow in a direction from the distal end **60** to the proximal end **62.** The resiliently elastic elements **101** bias the flow controller **69** against the sealing lip **99** when no liquid flow is present. In this manner, the flow controller **69** and the sealing lip **99** define a seal to prevent liquid from dripping between the flow controller **69** and the sealing lip **99.** Thus, the fill adapter **32** may be removed from the syringe **12a** without spilling any liquid that may remain in the fill adapter **32.** In some examples, the fill adapter **32** may be removably or non-removably connected to a cap **122** such that removal of the cap **122** from the syringe **12a** also removes the fill adapter **32.**

During a vacuum filling procedure, such as when an end wall **30** of the syringe **12a** is retracted in a proximal direction by a drive member of a liquid injector (not shown), vacuum generated within an interior volume **28** of the syringe **12a** pulls the flow controller **69** in the proximal direction against the restoring force of the resiliently elastic elements **101.** In other examples, liquid pressure due to a head height of liquid in the second container once the fill adapter **32** is connected to the second container will push the flow controller **69** in the proximal direction in the direction of arrow **A** in **FIG. 34B** against the restoring force of the resiliently elastic elements **101.** Movement of the flow controller **69** in the proximal direction unseats it from the sealing lip **99,** thereby opening a gap **79** between the inner circumference of the sealing lip **99** and an outer circumference of the flow controller **69,** such as shown in **FIG. 34B****.** The flow controller **69** may have a curved distal surface that is configured to divert liquid flowing onto the flow controller **69** in a radially outward direction toward the gap **79.** In this manner, liquid can flow into the interior volume **28** of the syringe **12a** through the gap **79.** As described herein, due to the characteristics of the Coand 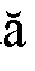 effect, liquid will be attracted to the angled surface of the angled portion **63** of the fill adapter **32** and will continue flowing down the inner sidewall of the syringe **12a.** This flow along the inside surface of the syringe **12a** helps to reduce turbulence as the liquid fills the syringe **12a,** which aides in reducing air bubbles from forming as the syringe **12a** is filled.

Proximal movement of the flow controller **69** in the direction of arrow **A** in **FIG. 34B** can be a function of the head pressure of the liquid in the second container, or the vacuum generated by a drive member of the liquid injector pulling the end wall **30** of the syringe **12a** (or a plunger **31** of the syringe **12b** in **FIG. 2B**) in the proximal direction, against the restoring force of the resiliently elastic members **101** acting in the distal direction. In this manner, the size of the gap **79** can be controlled to widen or narrow the gap **79** in order to optimize liquid flow due to the characteristics of the Coand 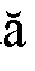 effect..

## Claims

1. A fill adapter (36) for delivery of a medical liquid to a container (12), the fill adapter (36) **characterized in** comprising:
a body (56) having a distal end (60), a proximal end (62), and a central bore (61) extending between the distal end (60) and the proximal end (62) along a longitudinal axis, the central bore (61) having an angled portion (63) at the proximal end (62) of the body (56) such that a diameter of the central portion increases at the angled portion (63) in a direction from the distal end (60) to the proximal end (62); and
a flow controller (69) comprising a curved distal surface (81) disposed within the central bore (61) at a distal end of the angled portion (63) such that a gap (79) is formed between an outer surface of the flow controller (69) and an inner surface of the central bore (61),
wherein at least a portion of the flow controller (69) is connected to the inner surface of the central bore (61) by one or more spokes (73) having a first end (75) connected to the inner surface of the central bore (61) and a second end (77) connected to the flow controller (69), and
wherein the flow controller (69) directs liquid flowing through the central bore (61) to flow through the gap (79) and along the angled portion (63) of the central bore (61) and down an inner sidewall (64) of the container (12) under a Coand 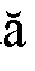 effect.

2. The fill adapter (36) of claim 1, wherein each of the one or more spokes (73) is resiliently elastic such that the flow controller (69) is movable in a direction of the longitudinal axis of the body (56) with the flow of the liquid.

3. The fill adapter (36) of claim 1, wherein the curved distal surface (81) of the flow controller (59) is convex, or
wherein preferably the curved distal surface (81) has a flow diverter (87) extending distally from a central portion of the curved distal surface (81), the flow diverter (87) shaped to direct liquid flowing through the central bore (61) in a radially outward direction toward the gap (79).

4. The fill adapter (36) of claim 1, wherein the flow controller (69) has a curved proximal surface (83), and wherein preferably the curved proximal surface (83) of the flow controller (69) is convex.

5. The fill adapter (36) of claim 1, wherein the body (56) has a flange (57) at the distal end (60), the flange (57) extending radially outward relative to an outer surface of the body (56).

6. The fill adapter (36) of claim 1, wherein at least a portion of the body (56) is configured to be removably received within an open distal end (23) of the container (12).

7. A fluid transfer assembly (200) with the fill adapter (36) according to claim 1, the fluid transfer assembly (200) comprising:
a syringe (12) for receiving a medical liquid therein, the syringe (12) comprising:
a proximal end (16), a distal end (18) having an open-ended syringe neck (22), and a sidewall (20) extending between the proximal end (16) and the distal end (18) along a longitudinal axis, the syringe (12) defining an interior volume (28) for receiving the medical liquid therein; and
wherein the fill adapter (36) received within the open-ended syringe neck (22).

8. The fluid transfer assembly (200) of claim 7, wherein an outer portion of the syringe neck (22) has a flange (57) extending around at least a portion of a circumference of the syringe neck (22).

9. The fluid transfer assembly (200) of claim 8, wherein at least a portion of the flange (57) is configured to engage a cap (122) for enclosing the distal end (18) of the syringe (12).

10. The fluid transfer assembly (200) of claim 7, wherein the syringe (12a) has a drive member engagement portion (134) protruding proximally from an end wall (30) enclosing the proximal end (16) and configured for engagement with a drive member of a fluid injector.

11. The fluid transfer assembly (200) of claim 7, wherein the sidewall (20) of the syringe (12a9 is flexible and rolls upon itself when acted upon by a drive member of a fluid injector such that an outer surface of the sidewall (20) is folded in a radially inward direction as the drive member is advanced from the proximal end (16) to the distal end (18), and wherein the outer surface of the sidewall (20) is unfolded in a radially outward direction as the drive member is retracted from the distal end (18) to the proximal end (16).

12. A fluid transfer assembly (200) with the fill adapter (36) according to claim 1, the fluid transfer assembly (200)_comprising:
a rolling diaphragm syringe (12a) for receiving a medical liquid therein, the syringe (12a) comprising:
a proximal end (16), a distal end (18) having an open-ended syringe neck (22), and a sidewall (20) extending between the proximal end (16) and the distal end (18) along a longitudinal axis, the syringe (12a) defining an interior volume for receiving the medical liquid therein;
wherein the fill adapter (36) is received within the open-ended syringe neck (22); and
a cap (122) secured to the syringe neck (22), the cap having a nozzle in liquid communication with the interior volume of the rolling diaphragm syringe (12a).

13. The fluid transfer assembly (200) of claim 12, wherein an outer portion of the syringe neck (22) has a flange (150) extending around at least a portion of a circumference of the syringe neck (22), and wherein the cap (122) has one or more tabs (142) configured to releasably engage at least a portion of the flange (150),
wherein preferably the one or more tabs (142) has a first end (142a) connected to the cap (122) and a second end (142b) extending proximally from the first end (142a), and wherein the second end (142b) is deflectable in a radially outward direction when the cap (122) contacts the flange (150) on the syringe neck 22).

## Patentansprüche

1. Fülladapter (36) zur Zufuhr einer medizinischen Flüssigkeit in einen Behälter (12), wobei der Fülladapter (36) **dadurch gekennzeichnet ist, dass** er Folgendes umfasst:
einen Körper (56) mit einem distalen Ende (60) und einem proximalen Ende (62) und einer sich zwischen dem distalen Ende (60) und dem proximalen Ende (62) entlang einer Längsachse erstreckenden mittleren Bohrung (61), wobei die mittlere Bohrung (61) einen abgewinkelten Abschnitt (63) an dem proximalen Ende (62) des Körpers (56) hat, so dass ein Durchmesser des mittleren Abschnitts an dem abgewinkelten Abschnitt (63) in einer Richtung von dem distalen Ende (60) zu dem proximalen Ende (62) zunimmt, und
einen Durchflussregler (69), der eine gekrümmte distale Fläche (81) umfasst, die in der mittleren Bohrung (61) an einem distalen Ende des abgewinkelten Abschnitts (63) angeordnet ist, so dass zwischen einer Außenfläche des Durchflussreglers (69) und einer Innenfläche der mittleren Bohrung (61) ein Spalt (79) ausgebildet ist,
wobei mindestens ein Abschnitt des Durchflussreglers (69) über eine oder mehrere Speichen (73) mit einem ersten Ende (75), das mit der Innenfläche der mittleren Bohrung (61) verbunden ist und einem zweiten Ende (77), das mit dem Durchflussregler (69) verbunden ist, mit der Innenfläche der mittleren Bohrung (61) verbunden ist, und
wobei der Durchflussregler (69) durch die mittlere Bohrung (61) fließende Flüssigkeit so lenkt, dass sie durch den Spalt (79) und gemäß einem Coand 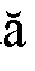 -Effekt entlang dem abgewinkelten Abschnitt (63) der mittleren Bohrung (61) und an einer inneren Seitenwand (64) des Behälters (12) hinunter strömt.

2. Fülladapter (36) nach Anspruch 1, wobei jede der einen oder der mehreren Speichen (73) federnd elastisch ist, so dass der Durchflussregler (69) in eine Richtung der Längsachse des Körpers (56) mit der Strömung der Flüssigkeit beweglich ist.

3. Fülladapter (36) nach Anspruch 1, wobei die gekrümmte distale Fläche (81) des Durchflussreglers (59) konvex ist oder
wobei vorzugsweise die gekrümmte distale Fläche (81) einen Strömungsumlenker (87) hat, der sich distal von einem mittleren Abschnitt der gekrümmten distalen Fläche (81) erstreckt, wobei der Strömungsumlenker (87) so gestaltet ist, dass er durch die mittlere Bohrung (61) strömende Flüssigkeit in eine radial nach außen gehende Richtung zu dem Spalt (79) hin lenkt.

4. Fülladapter (36) nach Anspruch 1, wobei der Durchflussregler (69) eine gekrümmte proximale Fläche (83) hat und wobei die gekrümmte proximale Fläche (83) des Durchflussreglers (69) vorzugsweise konvex ist.

5. Fülladapter (36) nach Anspruch 1, wobei der Körper (56) an dem distalen Ende (60) einen Flansch (57) hat, wobei sich der Flansch (57) bezüglich einer Außenfläche des Körpers (56) radial nach außen erstreckt.

6. Fülladapter (36) nach Anspruch 1, wobei mindestens ein Abschnitt des Körpers (56) dazu ausgestaltet ist, entfernbar in einem offenen distalen Ende (23) des Behälters (12) aufgenommen zu werden.

7. Fluidtransferanordnung (200) mit dem Fülladapter (36) nach Anspruch 1, wobei die Fluidtransferanordnung (200) Folgendes umfasst:
eine Spritze (12) zur Aufnahme einer medizinischen Flüssigkeit darin, wobei die Spritze (12) Folgendes umfasst:
ein proximales Ende (16), ein distales Ende (18), das einen Spritzenhals (22) mit offenem Ende hat, und eine Seitenwand (20), die sich zwischen dem proximalen Ende (16) und dem distalen Ende (18) entlang einer Längsachse erstreckt, wobei die Spritze (12) ein inneres Volumen (28) zur Aufnahme der medizinischen Flüssigkeit darin definiert, und
wobei der Fülladapter (36) in dem Spritzenhals (22) mit dem offenen Ende aufgenommen ist.

8. Fluidtransferanordnung (200) nach Anspruch 7, wobei ein äußerer Abschnitt des Spritzenhalses (22) einen Flansch (57) hat, der sich um mindestens einen Abschnitt eines Umfangs des Spritzenhalses (22) herum erstreckt.

9. Fluidtransferanordnung (200) nach Anspruch 8, wobei mindestens ein Abschnitt des Flanschs (57) dazu ausgestaltet ist, eine Kappe (122) zum Umschließen des distalen Endes (18) der Spritze (12) in Eingriff zu nehmen.

10. Fluidtransferanordnung (200) nach Anspruch 7, wobei die Spritze (12a) einen Antriebsglied-Eingriffsabschnitt (134) hat, der proximal von einer das proximale Ende (16) umschließenden Endwand (30) vorragt und zum Eingriff mit einem Antriebsglied eines Fluidinjektors ausgestaltet ist.

11. Fluidtransferanordnung (200) nach Anspruch 7, wobei die Seitenwand (20) der Spritze (12a) flexibel ist und sich aufrollt, wenn sie von einem Antriebsglied eines Fluidinjektors beaufschlagt wird, so dass eine Außenfläche der Seitenwand (20) in eine radial nach innen gehende Richtung gefaltet wird, während das Antriebsglied von dem proximalen Ende (16) zu dem distalen Ende (18) vorgeschoben wird, und wobei die Außenfläche der Seitenwand (20) in eine radial nach außen gehende Richtung entfaltet wird, während das Antriebsglied von dem distalen Ende (18) zu dem proximalen Ende (16) zurückgezogen wird.

12. Fluidtransferanordnung (200) mit dem Fülladapter (36) nach Anspruch 1, wobei die Fluidtransferanordnung (200) Folgendes umfasst:
eine Rollmembranspritze (12a) zur Aufnahme einer medizinischen Flüssigkeit darin, wobei die Spritze (12a) Folgendes umfasst:
ein proximales Ende (16), ein distales Ende (18), das einen Spritzenhals (22) mit offenem Ende hat, und eine Seitenwand (20), die sich zwischen dem proximalen Ende (16) und dem distalen Ende (18) entlang einer Längsachse erstreckt, wobei die Spritze (12a) ein inneres Volumen zur Aufnahme der medizinischen Flüssigkeit darin definiert,
wobei der Fülladapter (36) in dem Spritzenhals (22) mit dem offenen Ende aufgenommen ist und
eine an dem Spritzenhals (22) befestigte Kappe (122), wobei die Kappe eine Düse in Flüssigkeitsverbindung mit dem inneren Volumen der Rollmembranspritze (12a) hat.

13. Fluidtransferanordnung (200) nach Anspruch 12, wobei ein äußerer Abschnitt des Spritzenhalses (22) einen Flansch (150) hat, der sich um mindestens einen Abschnitt eines Umfangs des Spritzenhalses (22) herum erstreckt, und wobei die Kappe (122) eine oder mehrere Laschen (142) hat, die dazu ausgestaltet sind, mindestens einen Abschnitt des Flanschs (150) freigebbar in Eingriff zu nehmen, wobei vorzugsweise die eine oder die mehreren Laschen (142) ein mit der Kappe (122) verbundenes erstes Ende (142a) und ein sich proximal von dem ersten Ende (142a) erstreckendes zweites Ende (142b) haben und wobei das zweite Ende (142b) in eine radial nach außen gehende Richtung ablenkbar ist, wenn die Kappe (122) den Flansch (150) an dem Spritzenhals (22) kontaktiert.

## Revendications

1. Adaptateur de remplissage (36) pour la distribution d'un liquide médical dans un récipient (12), l'adaptateur de remplissage (36) étant **caractérisé en ce qu'**il comprend :
un corps (56) ayant une extrémité distale (60), une extrémité proximale (62), et un alésage central (61) s'étendant entre l'extrémité distale (60) et l'extrémité proximale (62) le long d'un axe longitudinal, l'alésage central (61) ayant une portion inclinée (63) au niveau de l'extrémité proximale (62) du corps (56) de telle sorte qu'un diamètre de la portion centrale augmente au niveau de la portion inclinée (63) dans une direction allant de l'extrémité distale (60) à l'extrémité proximale (62) ; et
un régulateur d'écoulement (69) comprenant une surface distale courbe (81) disposée à l'intérieur de l'alésage central (61) au niveau d'une extrémité distale de la portion inclinée (63) de telle sorte qu'un espace (79) soit formé entre une surface extérieure du régulateur d'écoulement (69) et une surface intérieure de l'alésage central (61),
au moins une portion du régulateur d'écoulement (69) étant connectée à la surface intérieure de l'alésage central (61) par un ou plusieurs rayons (73) ayant une première extrémité (75) connectée à la surface intérieure de l'alésage central (61) et une deuxième extrémité (77) connectée au régulateur d'écoulement (69), et
le régulateur d'écoulement (69) dirigeant du liquide s'écoulant à travers l'alésage central (61) de manière à ce qu'il s'écoule à travers l'espace (79) et le long de la portion inclinée (63) de l'alésage central (61) et descende le long d'une paroi latérale intérieure (64) du récipient (12) en raison d'un effet Coand 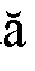 .

2. Adaptateur de remplissage (36) selon la revendication 1, dans lequel chacun du ou des rayons (73) est élastiquement flexible de telle sorte que le régulateur d'écoulement (69) puisse être déplacé dans une direction de l'axe longitudinal du corps (56) avec l'écoulement du liquide.

3. Adaptateur de remplissage (36) selon la revendication 1, dans lequel la surface distale courbe (81) du régulateur d'écoulement (59) est convexe, ou
dans lequel de préférence, la surface distale courbe (81) présente un déflecteur d'écoulement (87) s'étendant dans la direction distale depuis une portion centrale de la surface distale courbe (81), le déflecteur d'écoulement (87) étant formé pour diriger le liquide s'écoulant à travers l'alésage central (61) dans une direction radialement vers l'extérieur vers l'espace (79).

4. Adaptateur de remplissage (36) selon la revendication 1, dans lequel le régulateur d'écoulement (69) présente une surface proximale courbe (83) et dans lequel, de préférence, la surface proximale courbe (83) du régulateur d'écoulement (69) est convexe.

5. Adaptateur de remplissage (36) selon la revendication 1, dans lequel le corps (56) présente une bride (57) au niveau de l'extrémité distale (60), la bride (57) s'étendant radialement vers l'extérieur par rapport à une surface extérieure du corps (56).

6. Adaptateur de remplissage (36) selon la revendication 1, dans lequel au moins une portion du corps (56) est configurée pour être reçue de manière amovible à l'intérieur d'une extrémité distale ouverte (23) du récipient (12).

7. Ensemble de transfert de fluide (200) avec l'adaptateur de remplissage (36) selon la revendication 1, l'ensemble de transfert de fluide (200) comprenant :
une seringue (12) pour recevoir un liquide médical dans celle-ci, la seringue (12) comprenant :
une extrémité proximale (16), une extrémité distale (18) ayant un col de seringue à extrémité ouverte (22), et une paroi latérale (20) s'étendant entre l'extrémité proximale (16) et l'extrémité distale (18) le long d'un axe longitudinal, la seringue (12) définissant un volume intérieur (28) pour recevoir le liquide médical dans celui-ci ; et
l'adaptateur de remplissage (36) étant reçu à l'intérieur du col de seringue à extrémité ouverte (22) .

8. Ensemble de transfert de fluide (200) selon la revendication 7, dans lequel une portion extérieure du col de seringue (22) présente une bride (57) s'étendant autour d'au moins une portion d'une circonférence du col de seringue (22) .

9. Ensemble de transfert de fluide (200) selon la revendication 8, dans lequel au moins une portion de la bride (57) est configurée pour venir en prise avec un capuchon (122) pour envelopper l'extrémité distale (18) de la seringue (12).

10. Ensemble de transfert de fluide (200) selon la revendication 7, dans lequel la seringue (12a) présente une portion d'engagement d'organe d'entraînement (134) faisant saillie dans la direction proximale depuis une paroi d'extrémité (30) enveloppant l'extrémité proximale (16) et configurée pour venir en prise avec un organe d'entraînement d'un injecteur de fluide.

11. Ensemble de transfert de fluide (200) selon la revendication 7, dans lequel la paroi latérale (20) de la seringue (12a) est flexible et s'enroule sur elle-même lorsqu'un organe d'entraînement d'un injecteur de fluide agit sur celle-ci de telle sorte qu'une surface extérieure de la paroi latérale (20) soit repliée dans une direction radialement vers l'intérieur à mesure que l'organe d'entraînement est avancé depuis l'extrémité proximale (16) jusqu'à l'extrémité distale (18), et la surface extérieure de la paroi latérale (20) étant dépliée dans une direction radialement vers l'extérieur à mesure que l'organe d'entraînement est rétracté depuis l'extrémité distale (18) jusqu'à l'extrémité proximale (16).

12. Ensemble de transfert de fluide (200) avec l'adaptateur de remplissage (36) selon la revendication 1, l'ensemble de transfert de fluide (200) comprenant :
une seringue à diaphragme roulant (12a) pour recevoir un liquide médical dans celle-ci, la seringue (12a) comprenant :
une extrémité proximale (16), une extrémité distale (18) ayant un col de seringue à extrémité ouverte (22), et une paroi latérale (20) s'étendant entre l'extrémité proximale (16) et l'extrémité distale (18) le long d'un axe longitudinal, la seringue (12a) définissant un volume intérieur pour recevoir le liquide médical dans celui-ci ;
l'adaptateur de remplissage (36) étant reçu à l'intérieur du col de seringue à extrémité ouverte (22) ; et
un capuchon (122) fixé au col de seringue (22), le capuchon ayant une buse en communication fluidique avec le volume intérieur de la seringue à diaphragme roulant (12a).

13. Ensemble de transfert de fluide (200) selon la revendication 12, dans lequel une portion extérieure du col de seringue (22) présente une bride (150) s'étendant autour d'au moins une portion d'une circonférence du col de seringue (22), et dans lequel le capuchon (122) présente un ou plusieurs taquets (142) configurés pour venir en prise de manière amovible avec au moins une portion de la bride (150),
de préférence le ou les taquets (142) ayant une première extrémité (142a) connectée au capuchon (122) et une deuxième extrémité (142b) s'étendant dans la direction proximale depuis la première extrémité (142a), et la deuxième extrémité (142b) pouvant être déviée dans une direction radialement vers l'extérieur lorsque le capuchon (122) vient en contact avec la bride (150) sur le col de seringue (22).
